# EUROPEAN PATENT APPLICATION

(11) **EP 4 509 125 A1**
(43) Date of publication of application: **19.02.2025**
(21) Application number: 23787559.6
(22) Date of filing: 04.04.2023
(51) Int. Cl.: A61K 31/519, A61K 31/5377, A61P 43/00, A61P 37/02, A61P 29/00, A61P 11/06, A61P 17/06, A61P 19/02, A61P 9/10

(54) **NOVEL USE OF PYRAZOLOPYRIMIDINE COMPOUND**

(30) Priority: 12.04.2022 CN 202210376872
(71) Applicant: Hefei Youyuan Pharmaceutical Co., Ltd., Hefei, Anhui (CN)
(72) Inventor: FU, Yufeng, Bengbu, Anhui 233000 (CN)
(74) Representative: Abel & Imray LLP
(86) International application number: PCT/CN2023/086239
(87) International publication number: WO 2023/197914

(57) **Abstract**

Provided is novel use of a compound of formula (I) or a pharmaceutically acceptable salt, solvate, isomer, ester, acid, metabolite, or prodrug thereof in preventing or treating hypersensitivity or autoimmune inflammation-related disorder.

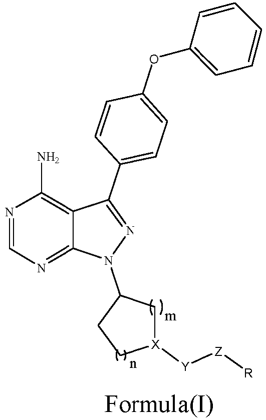

## Description

### Technical Field

The invention relates to a new use of RIPK2 kinase inhibitor compounds, and particularly the use and method of using these compounds to reduce or inhibit the activity of RIPK2 kinase, as well as prevent or treat hypersensitivity reactions or autoimmune inflammatory-related diseases in a subject.

### Background

In clinical practice, inflammatory bowel disease (IBD), including ulcerative colitics and Crohn's diseases, is a group of chronic recurrent non-specific intestinal inflammatory diseases. IBD tends to occur in young adults, characterized by rapid progression, significant destructiveness and the need for lifelong treatment. IBD drugs are limited and have poor efficacy. Patients often experience complications such as intestinal obstruction, bleeding, and canceration. 70-80% of patients require operative treatment, with high disability rates, poor prognosis and quality of life. Therefore, IBD is known as "green tumor". Currently, the traditional drugs for IBD treatment in China and overseas include amiosalicylic acid preparations, glucicirticoids, and immunosuppressants and the like. However, these drugs have disadvantages such as poor efficacy and significant side effects. In recent years, biological agents and small molecule drugs have been emerging constantly. However, their widespread clinical application is limited due to high costs and susceptibility to drug resistance. Thus, the research and development of innovative and effective treatment drugs is a pivotal scientific issue in the field of IBD research, with significant economic and social value. RIPK2 regulates proinflammatory signals mediated by NOD1 and NOD2, representing another emerging therapeutic target for autoimmune and inflammatory diseases. Small molecule compounds targeting RIPK2 can be applied to prevent or treat IBD (Hajime Honjo et al, RIPK2 as a New Therapeutic Target in Inflammatory Bowel Diseases, Front. Pharmacol., 2021, 12:650403. doi: 10.3389/fphar.2021.650403), hypersensitivity reactions or autoimmune inflammatory-related diseases (TapasMukherjee et al, NOD1 and NOD2 in inflammation, immunity and disease, Archives of Biochemistry and Biophysics, 2019, 670, 69-81).

In addition, studies have shown that RIPK2 regulates the high activation of proinflammatory signals mediated by NOD1 and NOD2 in patients with rheumatoid arthritis (RA) patients (RFO Franca et al, Expression and activity of NOD1 and NOD2/RIPK2 signalling in mononuclear cells from patients with rheumatoid arthritis, Scand J Rheumatol, 2016, 45(1):8-12). RA is a systemic autoimmune disease and a common chronic condition in clinical practice. It is characterized by persistent synovitis and systemic inflammation. The small joints are commonly affected regions in RA patients, with initial symptoms generally presenting as joint swelling, pain and stiffness. Synovitis is recurrent and persistent, which may lead to irreversible damage to the joint cartilage and bones of patients, significantly impact their normal activities and cause considerable inconvenience in their dairy life. When the disease develops seriously, it can lead to teratogenesis and disability in patients, and there is also a possibility of cardiovascular or other complications. There is currently no clear and systematical explanation of the pathogenic mechanisms of RA. Based on existing research, the widely accepted view is that the combined effects of various complex internal and external factors lead to abnormal behavior of synovial cells and chondrocytes in patients, and excessive production of numerous cytokines. These cytokines activate pivotal signal transduction pathways through various ways, triggering a series of inflammatory cascade reactions that ultimately result in damage to the joint synovial structure in patients, exhibiting typical clinical symptoms of RA. Due to the complexity of RA pathogenesis, there are various treatment options available. However, the ideal effect of completely curing RA have not been achieved to date, and people can only pursue long-term remission through short-term treatments. Different treatment measures play a significant role in RA therapy, but the most important among various effective treatments is drug therapy. Three types of drugs, including non-steroidal anti-inflammatory drugs, disease-modifying anti-rheumatic drugs, and glucocorticoids, once became classic drugs in RA therapy, and occupy important positions in the market. With the deepening research on RA, drugs with new mechanisms of action and better therapeutic effects have emerged as rising stars in the RA therapy market. In recent years, the application of chemical small molecule protein kinase inhibitors (PKi) in disease treatment has attracted the attention of many medical professionals, and its application in the RA therapy area has also become an urgent expectation for people.

### Summary of Invention

The series of compunds involved in this invention are currently primarily used for the treatment and/or inhibition of FLT3 kinase-related cancers, including various cancers such as leukemia. It has been discovered through inventive works that, this series of compounds may show significant therapeutic effects and application prospects for hypersensitivity reactions or autoimmune inflammatory-related diseases.

Therefore, the invention provides use of a compound of formula (I) or a pharmaceutically acceptable salt, solvate, isomer, ester, acid, metabolite, or prodrug thereof in the preparation of a medicament for treating or preventing hypersensitivity reactions or autoimmune inflammatory-related diseases. 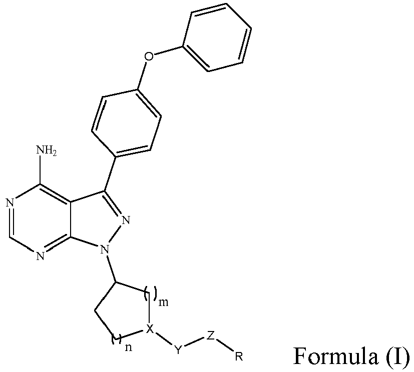 wherein:
m is selected from the integer of 1 or 2;
n is selected from the integer of 0-4;
X is N or CH, and when X is N, Y is a chemical bond; when X is CH, Y is NH;
Z is selected from CH₂, 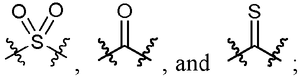
R is selected from the group consisting of amino, unsubstituted or substituted C1-C8 alkyl with carbon atom(s) being optionally substituted by 1-3 independent R1, unsubstituted or substituted C3-C8 cycloalkyl with carbon atom(s) being optionally substituted by 1-3 independent R1, unsubstituted or substituted C1-C8 haloalkyl with carbon atom(s) being optionally substituted by 1-3 independent R1, unsubstituted or substituted C1-C8 aminoalkyl with carbon atom(s) being optionally substituted by 1-3 independent R1 or heteroatom(s) being optionally substituted by R2, unsubstituted or substituted C1-C8 cyanoalkyl with carbon atom(s) being optionally substituted by 1-3 independent R1, unsubstituted or substituted C1-C8 hydroxyalkyl with carbon atom(s) being optionally substituted by 1-3 independent R1, unsubstituted or substituted C1-C8 alkoxy with carbon atom(s) being optionally substituted by 1-3 independent R1, unsubstituted or substituted C3-C8 heterocyclyl with carbon atom(s) being optionally substituted by 1-3 independent R1 or heteroatom(s) being optionally substituted by R2, unsubstituted or substituted aryl with carbon atom(s) being optionally substituted by 1-3 independent R1, unsubstituted or substituted heteroaryl with carbon atom(s) being optionally substituted by 1-3 independent R1 or heteroatom(s) being optionally substituted by R2, carbamoyl, unsubstituted or substituted C1-C8 alkylformyl with carbon atom(s) being optionally substituted by 1-3 independent R1, unsubstituted or substituted C3-C8 cycloalkylformyl with carbon atom(s) being optionally substituted by 1-3 independent R1, unsubstituted or substituted C3-C8 heterocyclyl formyl with carbon atom(s) being optionally substituted by 1-3 independent R1 or heteroatom(s) being optionally substituted by R2, unsubstituted or substituted arylformyl with carbon atom(s) being optionally substituted by 1-3 independent R1, unsubstituted or substituted C1-C8 alkylamino(C1-C8)alkyl with carbon atom(s) being optionally substituted by 1-3 independent R1, unsubstituted or substituted C1-C8 alkyl(C3-C6 cycloalkyl) with carbon atom(s) being optionally substituted by 1-3 independent R1, unsubstituted or substituted C3-C6 cycloalkyl(C1-C8 alkyl) with carbon atom(s) being optionally substituted by 1-3 independent R1, unsubstituted or substituted C1-C8 alkyl(C3-C6 heterocyclyl) with carbon atom(s) being optionally substituted by 1-3 independent R1 or heteroatom(s) being optionally substituted by R2, unsubstituted or substituted C3-C6 heterocyclyl(C1-C8 alkyl) with carbon atom(s) being optionally substituted by 1-3 independent R1 or heteroatom(s) being optionally substituted by R2, unsubstituted or substituted C1-C8 alkyl(aryl) with carbon atom(s) being optionally substituted by 1-3 independent R1, unsubstituted or substituted C1-C8 alkyl(heteroaryl) with carbon atom(s) being optionally substituted by 1-3 independent R1 or heteroatom(s) being optionally substituted by R2, and unsubstituted or substituted C1-C8 aminoalkyl(carbamoyl) with carbon atom(s) being optionally substituted by 1-3 independent R1;
R1 is selected from the group consisting of halogen, amino, nitro, cyano, hydroxyl, C1-C8 alkyl, C3-C8 cycloalkyl, C1-C8 alkoxy, aryl, heteroaryl with heteroatom(s) being optionally substituted by R2, C1-C8 alkoxy carbonyl, C1-C8 alkyl(heteroaryl) with heteroatom(s) being optionally substituted by R2, and C1-C8 alkyl(C3-C6 heterocyclyl) with heteroatom(s) being optionally substituted by R2;
R2 is selected from the group consisting of amino protecting group, C1-C8 alkyl, and C1-C8 alkoxy carbonyl. The amino protecting groups are independently selected from the group consisting of tert-butoxycarbonyl (Boc), benzyloxycarbonyl (Cbz), 9-fluorenylmethoxycarbonyl (FMOC), benzyl (Bn), and para-methoxyphenyl (PMP).

In some embodiments, n is preferably 1 or 2.

In some embodiments, Z is preferably

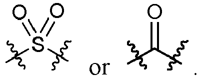

In some embodiments, R is preferably selected from the group consisting of substituted or unsubstituted C1-C6 alkyl (e.g., methyl, ethyl, n-propyl, isopropyl, n-butyl, isobutyl, tert-butyl, n-pentyl, isopentyl, and neopentyl, etc.), C1-C4 haloalkyl (e.g., chloromethyl, trifluoromethyl, trichloromethyl, chloroethyl, fluoroethyl, trifluoroethyl, chloropropyl, fluoropropyl, chlorobutyl, and fluorobutyl, etc.), C3-C6 cycloalkyl (e.g., cyclopropyl, cyclobutyl, cyclopentyl, and cyclohexyl, etc.), amino, carbamoyl, C1-C6 aminoalkyl (methyl, ethyl, propyl, butyl, and pentyl, etc., with any carbon atom being substituted by amino, and N being optionally substituted by R2 such as C1-4 alkyl and amino protecting group), heteroaryl (e.g., pyridyl, pyrimidyl, isoxazolyl, and benzodioxolyl, etc., with carbon atom(s) being optionally substituted by amino), C3-C6 heterocyclyl (e.g., piperazinyl, and piperidyl, etc., with N atom(s) being optionally substituted by C1-C4 alkyl and C1-C4 alkoxy carbonyl), aryl (e.g., phenyl, with carbon atom(s) being optionally substituted by C1-C4 alkoxy carbonyl and N-methylpiperazinylmethyl), C1-C4 cyanoalkyl (e.g., cyanomethyl, cyanoethyl, cyanopropyl, and cyanobutyl, etc.), di(C1-C4 alkyl)-N-(C1-C4)alkyl(e.g., dimethylaminomethyl, diethylaminomethyl, dimethylaminoethyl, diethylaminoethyl, dimethylaminopropyl, and dimethylaminobutyl, etc.), C1-C6 hydroxyalkyl (e.g., methyl, ethyl, propyl, butyl, and pentyl, etc., with any carbon atom being substituted by hydroxyl, and carbon atom(s) being optionally substituted by amino), C1-C4 alkyl(C3-C6 heterocyclyl)(e.g., morpholinylmethyl, piperazinylmethyl, and piperazinylethyl, etc., with N atom(s) being optionally substituted by C1-C4 alkyl), C1-C4 alkyl(C3-C6 cycloalkyl)(e.g., cyclopropylmethyl, cyclobutylmethyl, cyclopentylmethyl, cyclopropylethyl, cyclobutylethyl, cyclopentylethyl, cyclopropylpropyl, and cyclopropylbutyl, etc.), C1-C4 aminoalkyl(carbamoyl) (e.g., ethyl(carbamoyl), and propyl(carbamoyl), etc., with any carbon atom being substituted by amino), C1-C4 alkyl(heteroaryl) (e.g., imidazolylethyl, indolylethyl, imidazolylpropyl, and indolylpropyl, etc., with carbon atom(s) being optionally substituted by amino), and C1-C4 alkyl(aryl)(e.g., benzyl, phenylethyl, and phenylpropyl, etc., with carbon atom(s) being optionally substituted by amino or hydroxyl).

In some embodiments, m is 1, X is N, Y is a chemical bond, and the compound of the invention is represented by formula (II): 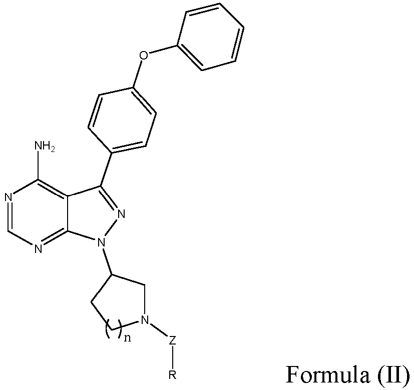 wherein, n, Z and R are defined as above.

In another embodiment, m is 2, X is N, Y is a chemical bond, and the compound of the invention is represented by formula (III): 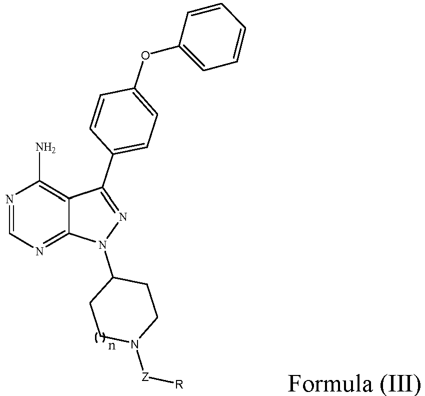 wherein, n, Z and R are defined as above. In this embodiment, n is preferably 1.

In another embodiment, m is 2, X is CH, Y is NH, and the compound of the invention is represented by formula (IV): 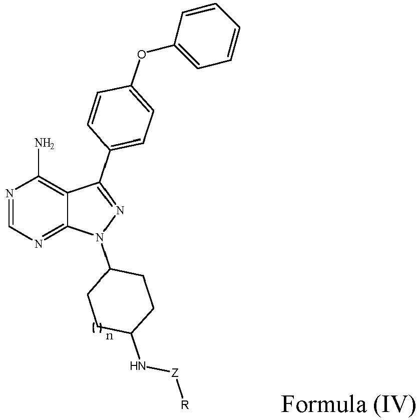 wherein, n, Z and R are defined as above. In this embodiment, n is preferably 1.

On the other hand, the invention relates to a use of the compound of formula (I), (II), (III), or (IV), or the pharmaceutically acceptable salt, solvate, isomer, ester, acid, metabolite, or prodrug thereof in the treatment or prevention of hypersensitivity reactions or autoimmune inflammatory-related diseases. In a preferred embodiment, the hypersensitivity reactions or autoimmune inflammatory-related diseases are selected from the group consisting of allergic rhinitis, asthma, atopic dermatitis, rheumatoid arthritis, multiple sclerosis, systemic lupus erythematosus, lupus nephritis, psoriasis, immune thrombocytopenic purpura, inflammatory bowel disease, colitis, chronic obstructive pulmonary disease, Sjögren's syndrome, pemphigus vulgaris, idiopathic plasmacytic lymphadenopathy, atherosclerosis, myocardial infarction, and thrombosis, and combinations thereof.

On another aspect, the invention relates to a use of the compound of formula (I), (II), (III), or (IV), or the pharmaceutically acceptable salt, solvate, isomer, ester, acid, metabolite, or prodrug thereof in reducing or inhibiting the activity of RIPK2 kinase *in vivo* or *in vitro.*

On another aspect, the invention relates to a use of the compound of formula (I), (II), (III), or (IV), or the pharmaceutically acceptable salt, solvate, isomer, ester, acid, metabolite, or prodrug thereof, or a pharmaceutical composition including the compound of formula (I), (II), (III), or (IV), in the preparation of a medicament for treating and/or inhibiting RIPK2 kinase activity related diseases.

### Description of Figures

Figure 1 shows the effect of compound 22 on the body weight of rats with inflammatory bowel disease.
Figure 2 shows the therapeutic effect of compound 22 on the colorectal ulcer area in rats with inflammatory bowel disease.
Figure 3 shows the comprehensive scores of compound 22 for colorectal ulcer in rats with inflammatory bowel disease.
Figure 4 shows the therapeutic effect of compound 22 on paw swelling in adjuvant-induced inflammatory model rats.
Figure 5 shows the scoring index of compound 22 for paw arthritis in adjuvant-induced inflammatory model rats.

### Detailed Description

### Terms

Unless otherwise defined, all scientific terms used herein have the same meaning as generally understood by skills in the art to which this claimed invention belongs.

Unless otherwise indicated, conventional methods of mass spectroscopy, NMR, HPLC, protein chemistry, biochemistry, recombinant DNA techniques and pharmacology that are within the skill of the art are employed in the invention. Unless specific definitions are provided, the nomenclature employed in connection with, and the laboratory procedures and techniques of, analytical chemistry, synthetic organic chemistry, and medicinal and pharmaceutical chemistry described herein are those known in the art. The foregoing techniques and procedures can be generally performed with conventional methods well known in the art and those as described in various general and more specific references that are cited and discussed throughout the present specification.

The "alkyl" refers to an aliphatic hydrocarbon group, which may be branched or straight alkyl. Depending on the structure, an alkyl group may be a monoradical or a diradical (i.e., an alkylene group). In the invention, the alkyl group is preferably a "lower alkyl" having 1 to 6 carbon atoms. Typical alkyl groups include, but are not limited to, methyl, ethyl, propyl, isopropyl, butyl, isobutyl, tert-butyl, pentyl, hexyl, and the like.

The "alkoxy" refers to a -O-alkyl group, where alkyl is as defined herein. Typical alkoxy groups include, but are not limited to, methoxy, ethoxy, propoxy, butoxy, pentyloxy, hexyloxy, and the like.

The "alkoxyalkyl" refers to an alkyl group as defined herein being substituted with alkoxy as defined herein.

The term "cyano" used herein refers to a -CN group.

The term "amino" refers to a -NH₂ group.

The term "alkylamino" refers to an amino substituent which is further substituted with one or two alkyl groups, specifically refers to the group -NRR', wherein R and R' are each independently selected from the group consisting of hydrogen or lower alkyl, with the proviso that -NRR' is not -NH₂. The term "arylalkylamino" refers to the group -NRR', wherein R is lower aryl, and R' is hydrogen, lower alkyl, aryl, or lower arylalkyl. The term "aminoalkyl" refers to an alkyl substituent further substituted with one or more amino groups. The term "hydroxyalkyl" or "hydroxylalkyl" refers to an alkyl substituent further substituted with one or more hydroxy groups. The term "cyanoalkyl" refers to an alkyl substituent further substituted with one or more cyano groups. The term "alkylcarbonyl" refers to a carbonyl group further substituted with an alkyl group. The term "alkylcarbonylalkyl" refers to an alkyl group further substituted with an alkylcarbonyl group. The term "alkoxycarbonyl" refers to a carbonyl group further substituted with an alkoxy group. The alkyl or aryl part of the groups alkylamino, aminoalkyl, hydroxyalkyl, cyanoalkyl, alkylcarbonyl, alkylcarbonylalkyl, and alkoxycarbonyl can be optionally substituted with one or more groups.

The "alkylaminoalkyl" refers to an alkyl group as defined herein being substituted with alkylamino as defined herein.

The term "aromatic" refers to a planar ring having a delocalized π-electron system containing 4n+2 π electrons, where n is an integer. Aromatic rings can be formed by five, six, seven, eight, nine, or more than nine atoms. Aromatics can be optionally substituted. The term "aromatic" includes both carbocyclic aryl (e.g., phenyl) and heterocyclic aryl (or "heteroaryl" or "heteroaromatic") groups (e.g., pyridine). The term includes monocyclic or fused-ring polycyclic (i.e., rings which share adjacent pairs of carbon atoms) groups.

As used herein, the term "aryl" refers to an aromatic ring wherein each of the atoms forming the ring is a carbon atom. Aryl rings can be formed by five, six, seven, eight, nine, or more than nine atoms. Aryl groups can be optionally substituted. Examples of aryl groups include, but are not limited to phenyl, naphthalenyl, phenanthrenyl, anthracenyl, fluorenyl, and indenyl. Depending on the structure, an aryl group can be a monoradical or a diradical (i.e., an arylene group).

The "alkyl(aryl)" or "arylalkyl" refers to an alkyl group as defined herein being substituted with aryl as defined herein. Nonrestrictive alkyl(aryl) includes benzyl, phenethyl and the like.

The term "cycloalkyl" refers to a monocyclic or polycyclic radical that contains only carbon and hydrogen. Cycloalkyl groups include groups having from 3 to 8 ring atoms. Depending on the structure, a cycloalkyl group can be a monoradical or a diradical (e.g., a cycloalkylene group). In the invention, the cycloalkyl group is preferably a cycloalkyl having 3 to 8 carbon atoms, and more preferably a "lower cycloalkyl" having 3 to 6 carbon atoms.

The "alkyl(cycloalkyl)" or "cycloalkylalkyl" refers to an alkyl group as defined herein being substituted with cycloalkyl as defined herein. Non-limiting examples of alkyl(cycloalkylalkyl) include cyclopropylmethyl, cyclobutylmethyl, cyclopentylmethyl, cyclohexylmethyl, and the like.

The term "heteroalkyl" used herein refers to an alkyl as defined herein with one or more chain backbone atom(s) being heteroatoms, such as oxygen, nitrogen, sulfur, silicon, phosphorus or their combination. The heteroatoms (one or more) may locate at any position within the heteroalkyl or at the position where the heteroalkyl is connected with the rest of the molecule.

The term "heteroaryl" refers to an aryl group that includes one or more ring heterocyclic atoms selected from nitrogen, oxygen, and sulfur. An N-containing "heteroaryl" moiety refers to an aromatic group in which at least one of the skeletal atoms of the ring is a nitrogen atom. Depending on the structure, the heteroaryl group may be a monoradical or a diradical (i.e., a heteroarylene group). Examples of heteroaryl groups include, but are not limited to pyridinyl, imidazolyl, pyrimidinyl, pyrazolyl, triazolyl, pyrazinyl, tetrazolyl, furyl, thienyl, isoxazolyl, thiazolyl, oxazolyl, isothiazolyl, pyrrolyl, quinolinyl, isoquinolinyl, indolyl, benzimidazolyl, benzofuryl, indazolyl, indolizinyl, phthalazinyl, pyridazinyl, isoindolyl, pteridinyl, purinyl, oxadiazolyl, thiadiazolyl, furazanyl, benzofurazanyl, benzothiophenyl, benzothiazolyl, benzoxazolyl, quinazolinyl, naphthyridinyl, furopyridinyl, and the like.

As used herein, the term "heterocycloalkyl" refers to a non-aromatic ring wherein one or more atoms forming the ring is a heteroatom selected from the group consisting of nitrogen, oxygen and sulfur. Heterocycloalkyl rings can formed by three, four, five, six, seven, eight, nine, or more than nine atoms. Heterocycloalkyl rings can be optionally substituted. Examples of heterocycloalkyls include, but are not limited to, lactams, lactones, cyclic imides, cyclic thioimides, cyclic carbamates, tetrahydrothiopyran, 4H-pyran, tetrahydropyran, piperidine, 1,3-dioxin, 1,3-dioxane, 1,4-dioxin, 1,4-dioxane, piperazine, 1,3-oxathiane, 1,4-oxathiin, 1,4-oxathiane, tetrahydro-1,4-thiazine, 2H-1,2-oxazine, maleimide, succinimide, barbituric acid, thiobarbituric acid, dioxopiperazine, hydantoin, dihydrouracil, morpholine, trioxane, hexahydro-1,3,5-triazine, tetrahydrothiophene, tetrahydrofuran, pyrroline, pyrrolidine, imidazolidine, pyrrolidone, pyrazoline, pyrazolidine, imidazoline, imidazolidine, 1,3-dioxole, 1,3-dioxolane, 1,3-dithiole, 1,3-dithiolane, isoxazoline, isoxazolidine, oxazoline, oxazolidine, oxazolidinone, thiazoline, thiazolidine, and 1,3-oxathiolane. Depending on the structure, a heterocycloalkyl group can be a monoradical or a diradical (i.e., a heterocycloalkylene group).

The "alkyl(heteroaryl)" or "heterarylalkyl" refers to an alkyl group as defined herein being substiututed with heteroaryl as defined herein.

The "alkyl(heterocycloalkyl)" or "heterocycloalkylalkyl" refers to an alkyl group as defined herein being substituted with heterocycloalkyl as defined herein.

The term "halo" or "halogen" refers to fluoro, chloro, bromo and iodo.

The terms "haloalkyl", "haloalkoxy" and "haloheteroalkyl" include alkyl, alkoxy or heteroalkyl structures in which at least one hydrogen is replaced with a halogen atom. In certain embodiments in which two or more hydrogen atoms are replaced with halogen atoms, the halogen atoms are the same or different from each other.

The term "acyl" refers to a monovalent atomic radical remaining after removal of the hydroxyl group from an organic or inorganic oxyacid, represented by a general formula of R-M(O)-, wherein M is usually C.

The term "carbonyl" is an organic functional group (C=O) formed by carbon atom and oxygen atom through a double bond linkage.

The term "substituted" refers to the mentioned group capable of being substituted by one or more additional groups, earch of which is individually and independently selected from the group consisting of alkyl, cycloalkyl, aryl, heteroaryl, hydroxyl, alkoxy, cyano, halogen, acylamino, nitro, haloalkyl, amino, alkoxycarbonyl, alkyl(heteroaryl), alkyl (heterocycloalkyl), and the like.

The terms kinase "inhibition", "inhibitory" or "inhibitor" used herein refer to the inhibition of phosphotransferase activity.

A "metabolite" of a compound disclosed herein is a derivative of that compound that is formed when the compound is metabolized. The term "active metabolite" refers to a biologically active derivative of a compound that is formed when the compound is metabolized. The term "metabolized" as used herein, refers to the sum of the processes (including, but not limited to, hydrolysis reactions and reactions catalyzed by enzymes, such as, oxidation reactions) by which a particular substance is changed by an organism. Thus, enzymes may produce specific structural alterations to a compound. For example, cytochrome P450 catalyzes a variety of oxidative and reductive reactions while uridine diphosphate glucuronyl transferases catalyze the transfer of an activated glucuronic acid molecule to aromatic alcohol, aliphatic alcohol, carboxylic acid, amine and free sulfhydryl group. Further information on metabolism may be obtained from The Pharmacological Basis of Therapeutics, 9th Edition, McGraw-Hill (1996). Metabolites of the compounds disclosed herein can be identified either by administration of compounds to a host and analysis of tissue samples from the host, or by incubation of compounds with hepatic cells *in vitro* and analysis of the resulting compounds. Both methods are well known in the art. In some embodiments, metabolites of a compound are formed by oxidative processes and correspond to the corresponding hydroxy-containing compound. In some embodiments, a compound is metabolized to pharmacologically active metabolite. The term "modulate" as used herein, means to interact with a target either directly or indirectly so as to alter the activity of the target, including, by way of example only, to enhance the activity of the target, to inhibit the activity of the target, to limit the activity of the target, or to extend the activity of the target.

The term "prodrug" includes compounds with components that can be metabolized in the body. Generally, prodrugs are metabolized into active drugs in the body through esterase or other mechanisms. The examples of prodrugs and their uses are well known in the field (referred such as Berge et al (1977) "Pharmaceutical Salts", J. Pharm. Sci. 66:1-19). The prodrugs may be prepared in situ during the final isolation and purification of the compound, or by separately reacting the purified compound in its free acid or hydroxyl with a suitable esterifying agent, respectively. Hydroxyl can be transformed into esters through treatment with carboxylic acid. Examples of prodrug components include substituted and unsubstituted, branched-chain or unbranched-chain lower alkyl ester components (such as propionic ester), lower alkenyl ester, di-lower alkyl-amino lower alkyl ester (such as dimethylaminoethyl ester), acyl amino lower alkyl ester (such as acetoxymethyl ester), acyloxy lower alkyl ester (such as pentanoyloxy methyl ester), aryl ester (phenyl ester), aryl lower alkyl ester (such as benzyl ester), substituted (such as being substituted by methyl, halogen, or methoxyl substituents) aryl and aryl lower alkyl ester, amide, lower alkyl amide, dilower alkyl amide and hydroxyl amide. The preferred prodrug components are propionic ester and acyl ester. Prodrugs that are transformed into active forms in the body through other mechanisms are also included. In some aspects, the compounds of this invention are prodrugs of any formula herein.

The term "isomer" refers to compounds with identical chemical compositions but differing spatial arrangements of atoms or groups, including diastereoisomers, enantiomers, regioisomers, structural isomers, rotamers, tautomers, etc. For compounds with one or more stereogenic centers, such as chiral compounds, enantiomerically enriched compounds, racemate, or mixtures of diastereoisomers can be used to implement the methods of this invention.

The term "target protein" used herein refers to a protein molecular or part of a protein that can be bound by a selective binding compound. In some embodiments, target protein is RIPK2.

As used herein, IC₅₀ refers to an amount, concentration or dosage of a particular test compound that achieves a 50% inhibition of a maximal response, in an assay that measures such response.

As used herein, EC₅₀ refers to a dosage, concentration or amount of a test compound that elicits a dose-dependent response at 50% of maximal expression of a particular response that is induced, provoked or potentiated by the particular test compound.

### RIPK2 kinase inhibitors of the invention

The RIPK2 kinase inhibitors of the invention include a compound of formula (I) or a pharmaceutically acceptable salt, solvate, isomer, ester, acid, metabolite, or prodrug thereof: 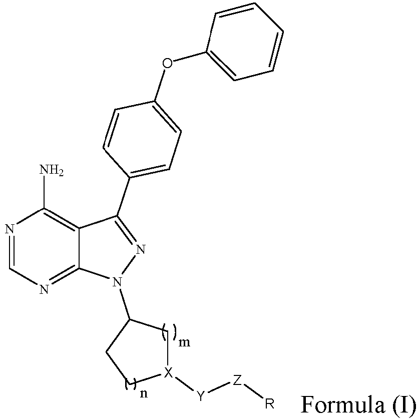 wherein:
m is selected from the integer of 1 or 2;
n is selected from the integer of 0-4;
X is N or CH, and when X is N, Y is chemical bond; when X is CH, Y is NH;
Z is selected from CH₂, 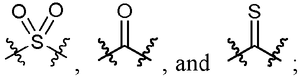
R is selected from the group consisting of amino, unsubstituted or substituted C1-C8 alkyl with carbon atom(s) being optionally substituted by 1-3 independent R1, unsubstituted or substituted C3-C8 cycloalkyl with carbon atom(s) being optionally substituted by 1-3 independent R1, unsubstituted or substituted C1-C8 haloalkyl with carbon atom(s) being optionally substituted by 1-3 independent R1, unsubstituted or substituted C1-C8 aminoalkyl with carbon atom(s) being optionally substituted by 1-3 independent R1 or heteroatom(s) being optionally substituted by R2, unsubstituted or substituted C1-C8 cyanoalkyl with carbon atom(s) being optionally substituted by 1-3 independent R1, unsubstituted or substituted C1-C8 hydroxyalkyl with carbon atom(s) being optionally substituted by 1-3 independent R1, unsubstituted or substituted C1-C8 alkoxy with carbon atom(s) being optionally substituted by 1-3 independent R1, unsubstituted or substituted C3-C8 heterocyclyl with carbon atom(s) being optionally substituted by 1-3 independent R1 or heteroatom(s) being optionally substituted by R2, unsubstituted or substituted aryl with carbon atom(s) being optionally substituted by 1-3 independent R1, unsubstituted or substituted heteroaryl with carbon atom(s) being optionally substituted by 1-3 independent R1 or heteroatom(s) being optionally substituted by R2, carbamoyl, unsubstituted or substituted C1-C8 alkylformyl with carbon atom(s) being optionally substituted by 1-3 independent R1, unsubstituted or substituted C1-C8 cycloalkylformyl with carbon atom(s) being optionally substituted by 1-3 independent R1, unsubstituted or substituted C3-C8 heterocyclyl formyl with carbon atom(s) being optionally substituted by 1-3 independent R1 or heteroatom(s) being optionally substituted by R2, unsubstituted or substituted arylformyl with carbon atom(s) being optionally substituted by 1-3 independent R1, unsubstituted or substituted C1-C8 alkylamino(C1-C8)alkyl with carbon atom(s) being optionally substituted by 1-3 independent R1, unsubstituted or substituted C1-C8 alkyl(C3-C6 cycloalkyl) with carbon atom(s) being optionally substituted by 1-3 independent R1, unsubstituted or substituted C3-C6 cycloalkyl(C1-C8 alkyl) with carbon atom(s) being optionally substituted by 1-3 independent R1, unsubstituted or substituted C1-C8 alkyl(C3-C6 heterocyclyl) with carbon atom(s) being optionally substituted by 1-3 independent R1 or heteroatom(s) being optionally substituted by R2, unsubstituted or substituted C3-C6 heterocyclyl(C1-C8 alkyl) with carbon atom(s) being optionally substituted by 1-3 independent R1 or heteroatom(s) being optionally substituted by R2, unsubstituted or substituted C1-C8 alkyl(aryl) with carbon atom(s) being optionally substituted by 1-3 independent R1, unsubstituted or substituted C1-C8 alkyl(heteroaryl) with carbon atom(s) being optionally substituted by 1-3 independent R1 or heteroatom(s) being optionally substituted by R2, and unsubstituted or substituted C1-C8 aminoalkyl(carbamoyl) with carbon atom(s) being optionally substituted by 1-3 independent R1;
R1 is selected from the group consisting of halogen, amino, nitro, cyano, hydroxyl, C1-C8 alkyl, C3-C8 cycloalkyl, C1-C8 alkoxy, aryl, heteroaryl with heteroatom(s) being optionally substituted by R2, C1-C8 alkoxy carbonyl, C1-C8 alkyl(heteroaryl) with heteroatom(s) being optionally substituted by R2, and C1-C8 alkyl(C3-C6 heterocyclyl) with heteroatom(s) being optionally substituted by R2;
R2 is selected from the group consisting of amino protecting group, C1-C8 alkyl, and C1-C8 alkoxy carbonyl. The amino protecting groups are independently selected from the group consisting of tert-butoxycarbonyl (Boc), benzyloxycarbonyl (Cbz), 9-fluorenylmethoxycarbonyl (FMOC), benzyl (Bn), and para-methoxyphenyl (PMP).

In some embodiments, n is preferably 1 or 2.

In some embodiments, Z is preferably

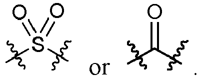

In some embodiments, R is preferably selected from the group consisting of substituted or unsubstituted C1-C6 alkyl (e.g., methyl, ethyl, n-propyl, isopropyl, n-butyl, isobutyl, tert-butyl, n-pentyl, isopentyl, and neopentyl, etc.), C1-C4 haloalkyl (e.g., chloromethyl, trifluoromethyl, trichloromethyl, chloroethyl, fluoroethyl, trifluoroethyl, chloropropyl, fluoropropyl, chlorobutyl, and fluorobutyl, etc.), C3-C6 cycloalkyl (e.g., cyclopropyl, cyclobutyl, cyclopentyl, and cyclohexyl, etc.), amino, carbamoyl, C1-C6 aminoalkyl (e.g., methyl, ethyl, propyl, butyl, and pentyl, etc., with any carbon atom being substituted by amino, and N being optionally substituted by R2 such as C1-4 alkyl and amino protecting group), heteroaryl (e.g., pyridyl, pyrimidyl, isoxazolyl, and benzodioxolyl, etc., with carbon atom(s) being optionally substituted by amino), C3-C6 heterocyclyl (e.g., piperazinyl, and piperidyl, etc., with N atom(s) being optionally substituted by C1-C4 alkyl and C1-C4 alkoxy carbonyl), aryl (e.g., phenyl, with carbon atom(s) being optionally substituted by C1-C4 alkoxy carbonyl and N-methyl(piperazinyl)methyl), C1-C4 cyanoalkyl (e.g., cyanomethyl, cyanoethyl, cyanopropyl, and cyanobutyl, etc.), di(C1-C4 alkyl)-N-(C1-C4)alkyl (e.g., dimethylaminomethyl, diethylaminomethyl, dimethylaminoethyl, diethylaminoethyl, dimethylaminopropyl, and dimethylaminobutyl, etc.), C1-C6 hydroxyalkyl (e.g., methyl, ethyl, propyl, butyl, and pentyl, etc., with any carbon atom being substituted by hydroxyl and carbon atom(s) being optionally substituted by amino), C1-C4 alkyl(C3-C6 heterocyclyl) (e.g., morpholinylmethyl, piperazinylmethyl, and piperazinylethyl, etc., with N atom(s) being optionally substituted by C1-C4 alkyl), C1-C4 alkyl(C3-C6 cycloalkyl) (e.g., cyclopropylmethyl, cyclobutylmethyl, cyclopentylmethyl, cyclopropylethyl, cyclobutylethyl, cyclopentylethyl, cyclopropylpropyl, and cyclopropylbutyl, etc.), C1-C4 aminoalkyl(carbamoyl) (e.g., ethyl(carbamoyl), and propyl(carbamoyl), etc., with any carbon atom being substituted by amino), C1-C4 alkyl(heteroaryl) (e.g., imidazolylethyl, indolylethyl, imidazolylpropyl, and indolylpropyl, etc., with carbon atom(s) being optionally substituted by amino), and C1-C4 alkyl(aryl) (e.g., benzyl, phenylethyl, and phenylpropyl, etc., with carbon atom(s) being optionally substituted by amino or hydroxyl).

In some embodiments, m is 1, X is N, Y is a chemical bond, and the compound of the invention is represented by formula (II): 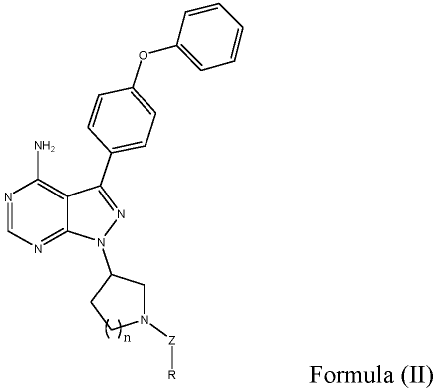 wherein, n, Z and R are defined as above. In this embodiment, n is preferably 1 or 2.

In another embodiment, m is 2, X is N, Y is a chemical bond, and the compound of the invention is represented by formula (III): 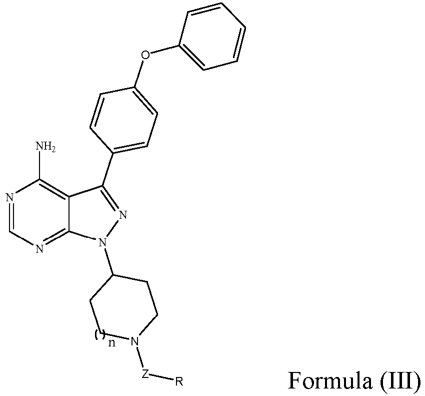 wherein, n, Z and R are defined as above. In this embodiment, n is preferably 1.

In another embodiment, m is 2, X is CH, Y is NH, and the compound of the invention is represented by formula (IV): 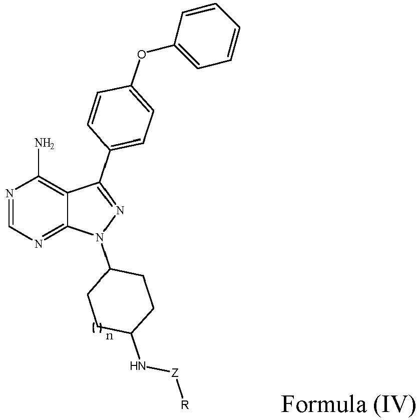 wherein, n, Z and R are defined as above. In this embodiment, n is preferably 1.

The chiral compounds involved in the invention may be of any configuration or racemic mixture.

On one hand, the following compounds are provided herein. The structures of these preferred compounds are shown in Table 1.

For each variable, any combination of the above groups is also under consideration in this specification. It can be understood that, the substituents and the substitution patterns of compounds provided herein may be selected by those skilled in the art, in order to provide compounds that are chemically stable and can be synthesized using known techniques in the field and those techniques described herein.

This specification describes a novel kinase inhibitor. Also described is the pharmaceutically acceptable salt, solvate, isomer, ester, acid, pharmaceutically active metabolite and prodrug of the compound.

In another or further embodiment, the compound described herein is administered to organisms in need and then metabolized in their bodies to produce metabolites. The produced metabolites are subsequently utilized to produce desired effects, including desired therapeutic effects.

The compound described herein can be prepared and/or be used as pharmaceutically acceptable salt. The types of pharmaceutically acceptable salts include, but are not limited to: (1) acid addition salts, formed by reacting the free base forms of compounds with pharmaceutically acceptable inorganic acids, such as hydrochloric acid, hydrobromic acid, sulfuric acid, nitric acid, phosphoric acid, meta phosphoric acid and the like; or formed by reacting with organic acids, such as acetic, propionic, hexanoic, cyclopentanepropionic, hydroxyacetic acid, pyruvic, lactic, malonic, malic, citric, succinic, maleic, tartaric, fumaric acid, trifluoroacetic, benzoic, 3-(4-hydroxybenzoyl)benzoic acid, cinnamic, mandelic, methanesulfonic, ethanesulfonic, 1,2-ethanedisulfonic, 2-hydroxyethanesulfonate, benzenesulfonic, toluenesulfonic, 4-methylbicyclo[2.2.2]oct-2-ene-1-carboxylic, 2-naphthalenesulfonic, tert-butyl acetic, glucoheptonic, 4,4'-methylenebis-(3-hydroxy-2-ene-1-formic), 3-phenylpropionic, trimethylacetic, dodecylsulfuric, gluconic, glutamic, salicylic, hydroxynaphthoic, stearic, muconic and the like; (2) base addition salts, formed when acidic protons in the parent compound are replaced by metal ions, such as alkali metal ions (for example lithium, sodium, potassium), alkaline earth metal ions (for example magnesium or calcium) or aluminium ions; or coordination with organic bases. Acceptable organic bases include ethanolamine, diehanolamine, triethanolamine, trimethylamine, N-mefhylglucamine, and the like. Acceptable inorganic bases include aluminum hydroxide, calcium hydroxide, potassium hydroxide, sodium carbonate, sodium hydroxide and the like.

The corresponding counterions of pharmaceutically acceptable salts can be analyzed and identified using various methods. The methods include, but are not limited to, ion exchange chromatography, ion chromatography, capillary electrophoresis, inductively coupled plasma, atomic absorption spectrum, mass spectrum or any combination thereof.

The salts can be recovered by using at least one of the following techniques: filtration, non-solvent precipitation followed by filtration, solvent evaporation, or lyophilization under the condition of aqueous solution.

The screening and characterization of pharmaceutically acceptable salts, polymorphs and/or solvates may be accomplished using a variety of techniques including, but not limited to, thermal analysis, X-ray diffraction, spectroscopy, microscopy, and elemental analysis. The various spectroscopic techniques used include, but are not limited to, Raman, FTIR, UVIS, and NMR (liquid and solid state). The various microscopy techniques include, but are not limited to, IR microscopy and Raman microscopy.

### Use of kinase inhibitors of the invention

The compound of formula (I), (II), (III), or (IV), or pharmaceutically acceptable salt, solvate, isomer, ester, acid, metabolite, or prodrug thereof, and the pharmaceutical composition including the same are called hereafter "the substances of the invention".

The substances of the invention can be used to treat or prevent hypersensitivity reactions or autoimmune inflammatory-related diseases. The hypersensitivity reactions and autoimmune inflammatory-related diseases are selected from the group consisting of: allergic rhinitis, asthma, atopic dermatitis, rheumatoid arthritis, multiple sclerosis, systemic lupus erythematosus, lupus nephritis, psoriasis, immune thrombocytopenic purpura, inflammatory bowel disease, colitis, chronic obstructive pulmonary disease, Sjögren's syndrome, pemphigus vulgaris, idiopathic plasmacytic lymphadenopathy, atherosclerosis, myocardial infarction, and thrombosis, and combinations thereof.

The preferred administration routes for warm-blooded animals, especially for humans, include enteral administration, such as nasal, oral, rectal, or especially oral, and parenteral administration, such as intravenously, intramuscularly, or subcutaneously. The doses of active ingredients may depend on the diseases and the genuses to be treated, age, weight and individual condition, individual pharmacokinetic data and administration route.

The compounds that can be used in combination with the compound of formula (I), (II), (III), or (IV) can be prepared and administered as described in this field.

In the embodiments of the invention, when treating the patients according to the invention, the dosage of administered drugs may depend on many factors, such as specific administration regimen, the types and severities of diseases and conditions, the uniqueness of patients or hosts requiring the treatment (such as body weight). However, based on the particular situation, including for example the specific drugs used, the administration routes, the conditions to be treated, and the patients or hosts to be treated, the dosage to be administrated can be routinely determined through established methods in this field. Generally, with respect to the dosage used in adult treatment, the dosage to be administrated typically ranges from 0.02 to 5000 mg/day, such as approximately 1 to 1500 mg/day. The required dosage can be conveniently administered as a single dose, or concurrently (or within a short timeframe), or as divided doses at appropriate intervals, such as two, three, four, or more divided doses per day. It can be understood by those skilled in the art that, while the above dosage ranges are given, the specific effective dosage may be appropriately adjusted according to the conditions of the patients under the diagnoses of doctors.

### Preparation of compounds

Compound of formula (I), (II), (III) or (IV) can be synthesized using standard synthetic techniques known to those skilled in the art or using methods known in the art in combination with methods described herein. Additionally, solvents, temperatures and other reaction conditions presented herein may vary according to techniques in the art.

In some embodiments, this specification provides the preparation methods and application methods of kinase inhibitor compounds described herein. In some embodiments, the compounds described herein can be synthesized using the following synthetic schemes. Methods similar to those described below can be used to synthesize compounds by using suitable optional starting materials.

The starting materials used for the synthesis of the compounds described herein can be synthesized or obtained from commercial sources. The compounds described herein and other related compounds with different substituents can be synthesized using techniques and materials known to those skilled in the art. In order to introducing various moieties into the molecules as provided herein, the reactions for the preparation of compounds as disclosed herein can be modified by appropriate reagents and conditions, as would be recognized by those skilled in the art.

The products of the reactions can be isolated and purified, if necessary, using conventional techniques, including, but not limited to, filtration, distillation, crystallization, chromatography and the like. Such products can be characterized using conventional methods, including physical constants and spectral data.

Using the synthesis methods described herein, the compounds disclosed herein are obtained with high yield and purity. The compounds prepared by the methods disclosed herein are purified through conventional methods known in the art, such as filtration, recrystallization, chromatography, distillation and combinations thereof.

Sites on the aromatic ring portion of the compound of formula (I), (II), (III) or (IV) may be liable to undergo various metabolic reactions. Thus, appropriate substiuent groups are introduced to the aromatic ring structure. Just as an example, halogens can reduce, diminish or eliminate this metabolic pathway.

### Examples

The following specific non-limiting examples should be interpreted as illustrative only, but not limiting the disclosure in any way. Although no further detailed description is required, it is believed that those skilled in the art can fully utilize the present disclosure based on the description herein.

Example compounds are shown in Table 1. The non-restrictive examples of the synthesis schemes for preparing compounds of formula (II) may refer to scheme I. The non-restrictive examples of the synthesis schemes for preparing compounds of formula (III) may refer to scheme II. The non-restrictive examples of the synthesis schemes for preparing compounds of formula (IV) may refer to scheme III.

**Table 1 Structures of example compounds**

| Ex. Comp. | Structures of Comp. | Ex. Comp. | Structures of Comp. |
|---|---|---|---|
| Compound 1 | 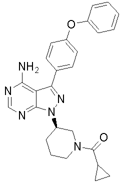 | Compound 2 | 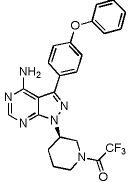 |
| Compound 3 | 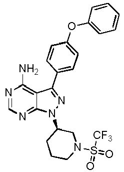 | Compound 4 | 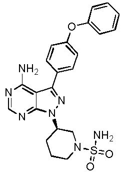 |
| Compound 5 | 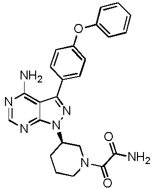 | Compound 6 | 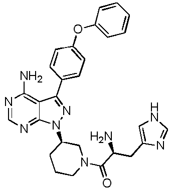 |
| Compound 7 | 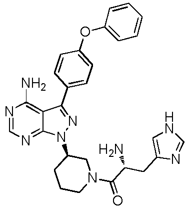 | Compound 8 | 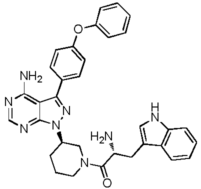 |
| Compound 9 | 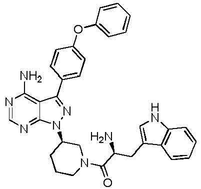 | Compound 10 | 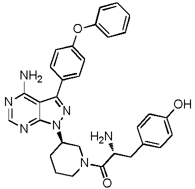 |
| Compound 11 | 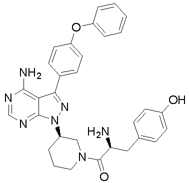 | Compound 12 | 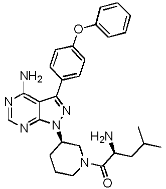 |
| Compound 13 | 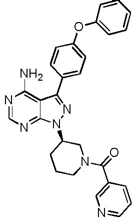 | Compound 14 | 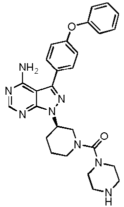 |
| Compound 15 | 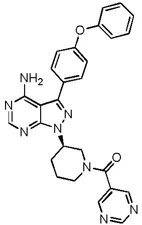 | Compound 16 | 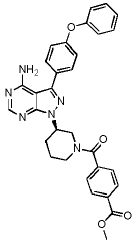 |
| Compound 17 | 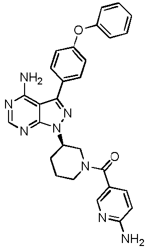 | Compound 18 | 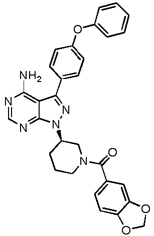 |
| Compound 19 | 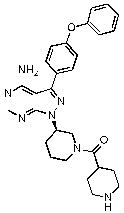 | Compound 20 | 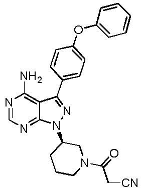 |
| Compound 21 | 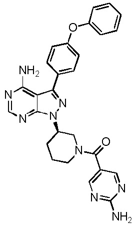 | Compound 22 | 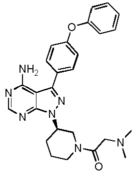 |
| Compound 23 | 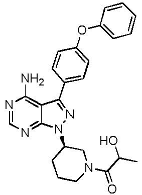 | Compound 24 | 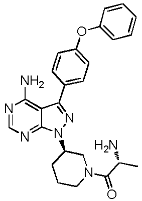 |
| Compound 25 | 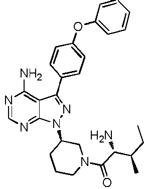 | Compound 26 | 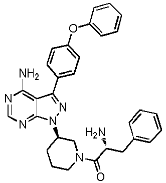 |
| Compound 27 | 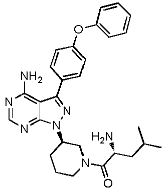 | Compound 28 | 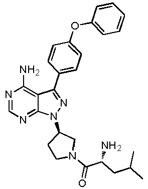 |
| Compound 29 | 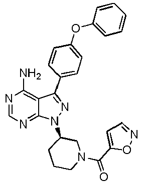 | Compound 30 | 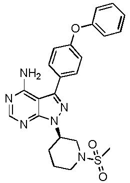 |
| Compound 31 | 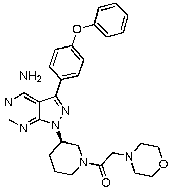 | Compound 32 | 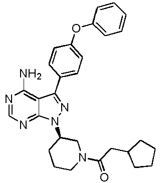 |
| Compound 33 | 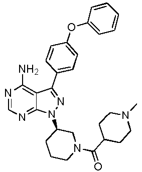 | Compound 34 | 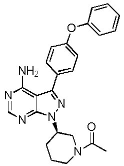 |
| Compound 35 | 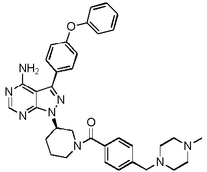 | Compound 36 | 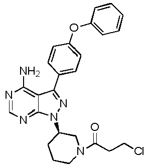 |
| Compound 37 | 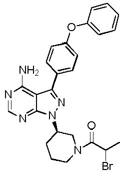 | Compound 38 | 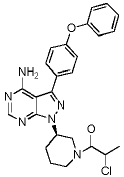 |
| Compound 39 | 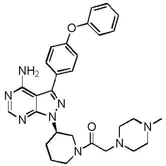 | Compound 40 | 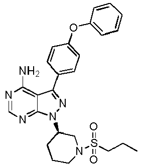 |
| Compound 41 | 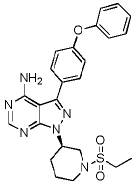 | Compound 42 | 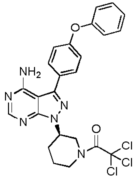 |
| Compound 43 | 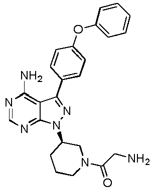 | Compound 44 | 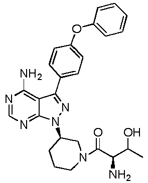 |
| Compound 45 | 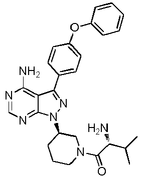 | Compound 46 | 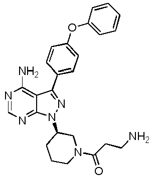 |
| Compound 47 | 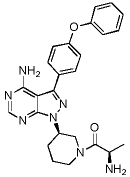 | Compound 48 | 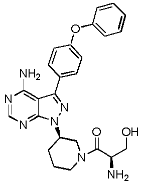 |
| Compound 49 | 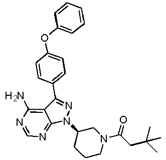 | Compound 50 | 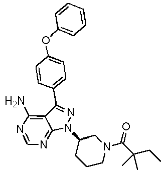 |
| Compound 51 | 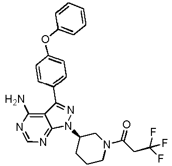 | Compound 52 | 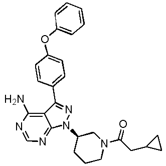 |
| Compound 53 | 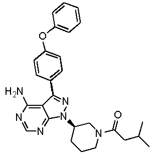 | Compound 54 | 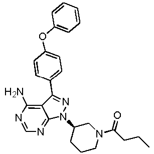 |
| Compound 55 | 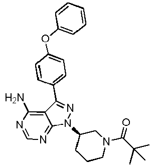 | Compound 56 | 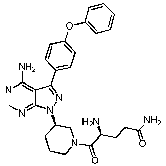 |
| Compound 57 | 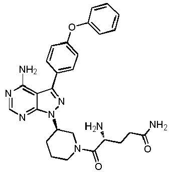 | Compound 58 | 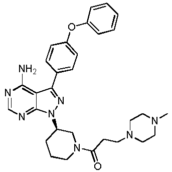 |
| Compound 59 | 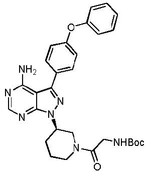 | Compound 60 | 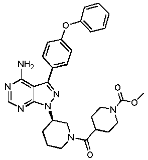 |
| Compound 61 | 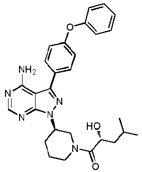 | Compound 62 | 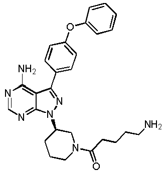 |
| Compound 63 | 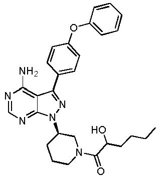 | Compound 64 | 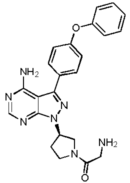 |
| Compound 65 | 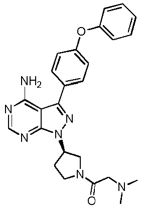 | Compound 66 | 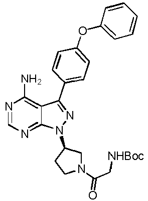 |
| Compound 67 | 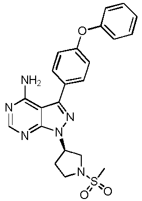 | Compound 68 | 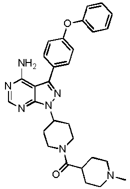 |
| Compound 69 | 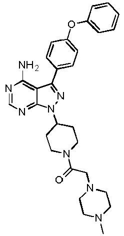 | Compound 70 | 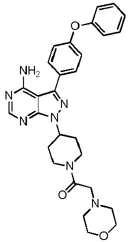 |
| Compound 71 | 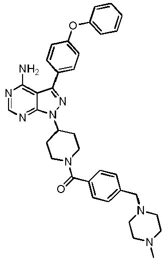 | Compound 72 | 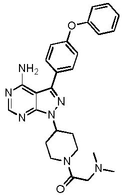 |
| Compound 73 | 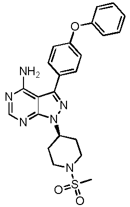 | Compound 74 | 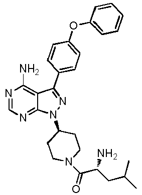 |
| Compound 75 | 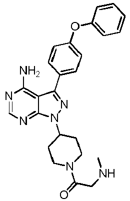 | Compound 76 | 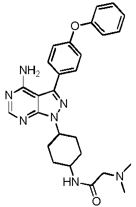 |
| Compound 77 | 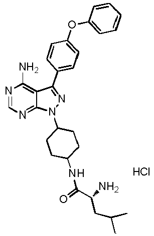 | Compound 78 | 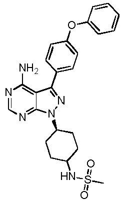 |
| Compound 79 | 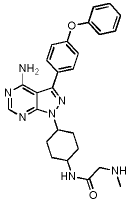 | Compound 80 | 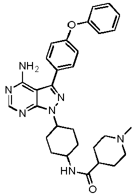 |
| Compound 81 | 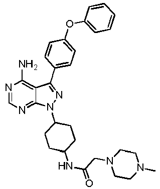 | Compound 82 | 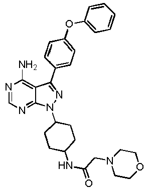 |
| Compound 83 | 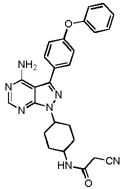 | Compound 84 | 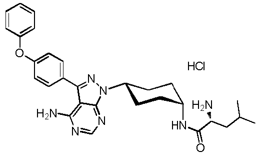 |
| Compound 85 | 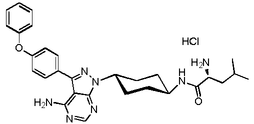 | Compound 86 | 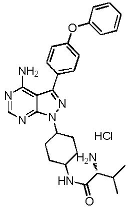 |
| Compound 87 | 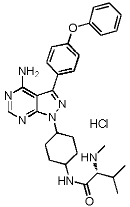 | Compound 88 | 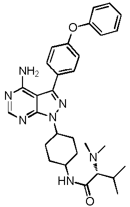 |
| Compound 89 | 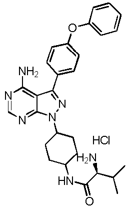 | Compound 90 | 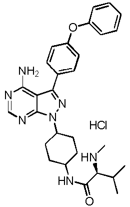 |
| Compound 91 | 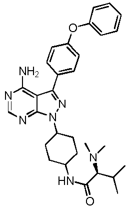 | Compound 92 | 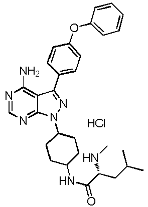 |
| Compound 93 | 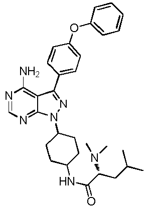 | Compound 94 | 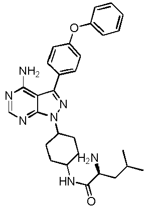 |
| Compound 95 | 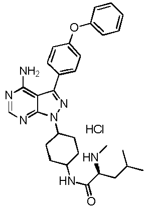 | Compound 96 | 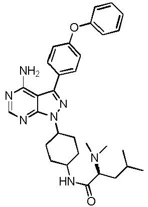 |
| Compound 97 | 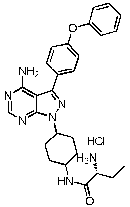 | Compound 98 | 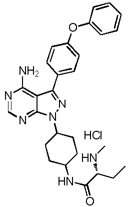 |
| Compound 99 | 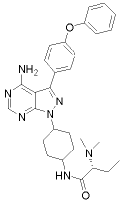 | Compound 100 | 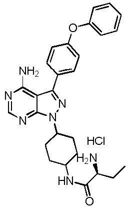 |
| Compound 101 | 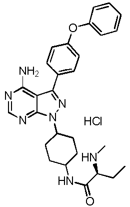 | Compound 102 | 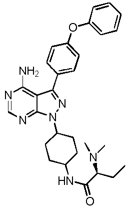 |
| Compound 103 | 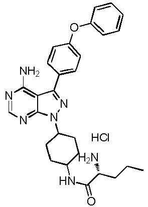 | Compound 104 | 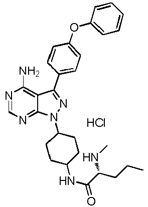 |
| Compound 105 | 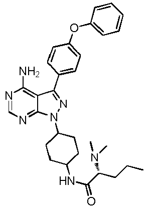 | Compound 106 | 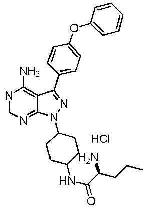 |
| Compound 107 | 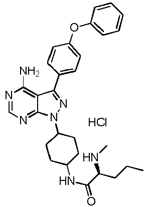 | Compound 108 | 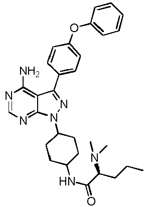 |
| Compound 109 | 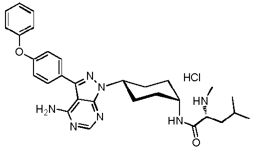 | Compound 110 | 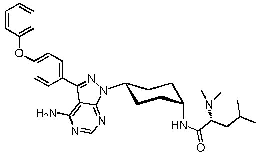 |
| Compound 111 | 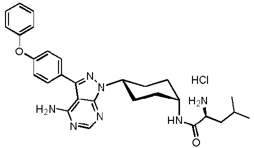 | Compound 112 | 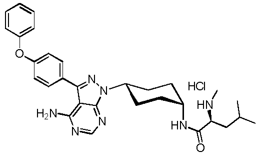 |
| Compound 113 | 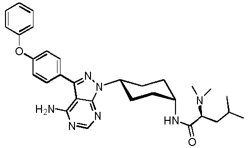 | Compound 114 | 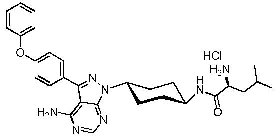 |
| Compound 115 | 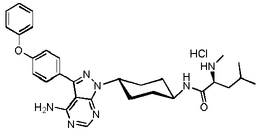 | Compound 116 | 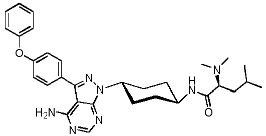 |
| Compound 117 | 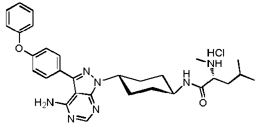 | Compound 118 | 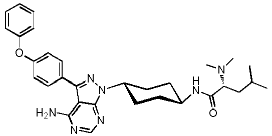 |
| Compound 119 | 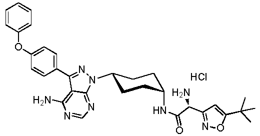 | Compound 120 | 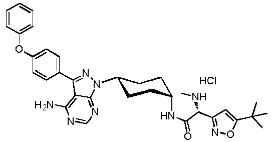 |
| Compound 121 | 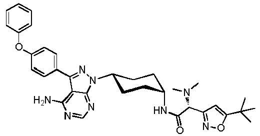 | Compound 122 | 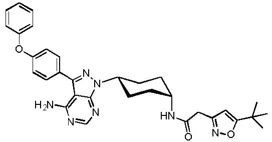 |
| Compound 123 | 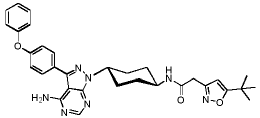 | Compound 124 | 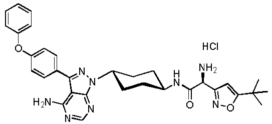 |
| Compound 125 | 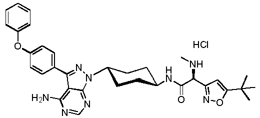 | Compound 126 | 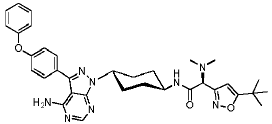 |

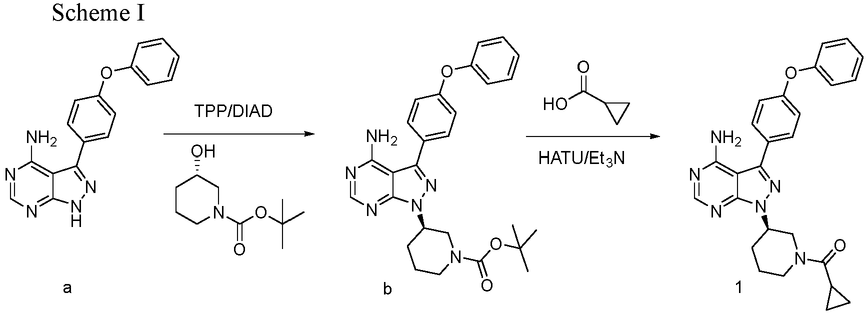

### Example 1

### Synthesis of compound 1, (R)-(3-(4-amino-(4-phenoxyphenyl)-1H-pyrazolo[3,4-d] pyrimidin-1-yl)piperidin-1-yl)(cyclopropyl)methanone

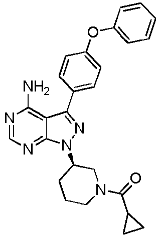

As shown in scheme I, using a to synthesize b; wherein, a could be prepared through methods known in the art or obtained commercially. When using a to synthesize b, (S)-N-tert-butoxycarbonyl-3-piperidinol was used to obtain intermediate b in R configuration. Intermediate b (0.1 g) was treated with solution of 4N HCl in dioxane (1 ml), stirred for 1 h at room temperature, and then the mixture was concentrated to yield a dry product of 0.08 g. The resultant was dissolved in 4 ml dichlormethane, and then diisopropylethylamine (0.2 mL), cyclopropanecarboxylic acid (0.03 g), and 2-(7-azobenzotriazole)-N,N,N',N'-tetramethylurea hexafluorophosphate HATU (0.15 g) were added in sequence. After stirring for 30 min at room temperature, 20 ml of dichlormethane was added for dilution, followed by sequential washing with water and saturated salt solution, drying over anhydrous Na₂SO₄, filtration, and vacuum drying. The residue was purified by column chromatography to yield compound 1 (0.03 g). Exact Mass (calculated value):454.21; MS(ESI) m/z(M+1)⁺: 455.22.

### Example 2

### Synthesis of compound 2, (R)-1-(3-(4-amino-(4-phenoxyphenyl)-1H-pyrazolo [3,4-d]pyrimidin-1-yl)piperidin-1-yl)-2,2,2-trifluoroethanone

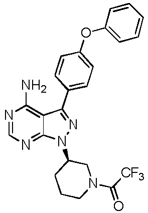

The synthesis of compound 2 was completed in the similar manner as described in example 1. Exact Mass (calculated value): 482.17; MS(ESI) m/z(M+1)⁺: 483.1722.

### Example 3

### Synthesis of compound 3, (R)-3-(4-phenoxyphenyl)-1-(1-((trifluoromethyl) sulfonyl)piperidin-3-yl)-1H-pyrazolo[3,4-d]pyrimidin-4-amine

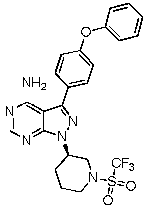

The synthesis of compound 3 was completed in the similar manner as described in example 1. Exact Mass (calculated value): 518.13; MS(ESI) m/z(M+1)⁺: 519.1315.

### Example 4

### Synthesis of compound 4, (R)-3-(4-amino-(4-phenoxyphenyl)-1H-pyrazolo [3,4-d]pyrimidin-1-yl)piperidin-1-sulfonamide

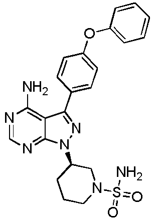

The synthesis of compound 4 was completed in the similar manner as described in example 1. Exact Mass (calculated value): 465.16; MS(ESI) m/z(M+1)⁺: 466.1615.

### Example 5

### Synthesis of compound 5, (R)-2-(3-(4-amino-(4-phenoxyphenyl)-1H-pyrazolo [3,4-d]pyrimidin-1-yl)piperidin-1-yl)-2-oxamide

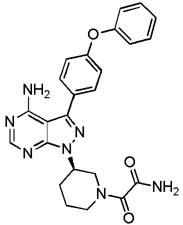

The synthesis of compound 5 was completed in the similar manner as described in example 1. Exact Mass (calculated value): 457.19; MS(ESI) m/z(M+1)⁺: 458.1915.

### Example 6

### Synthesis of compound 6, (S)-2-amino-1-((R)-3-(4-amino-(4-phenoxyphenyl)-1H-pyrazolo[3,4-d]pyrimidin-1-yl)piperidin-1-yl)-3-(1H-imidazol-4-yl)propan-1-one

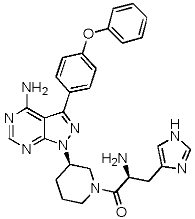

The synthesis of compound 6 was completed in the similar manner as described in example 1. Exact Mass (calculated value): 523.24; MS(ESI) m/z(M+1)⁺: 524.2413.

### Example 7

### Synthesis of compound 7, (R)-2-amino-1-((R)-3-(4-amino-(4-phenoxyphenyl)-1H-pyrazolo[3,4-d]pyrimidin-1-yl)piperidin-1-yl)-3-(1H-imidazol-4-yl) propan-1-one

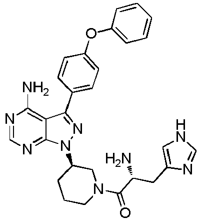

The synthesis of compound 7 was completed in the similar manner as described in example 1. Exact Mass (calculated value): 523.23; MS(ESI) m/z(M+1)⁺: 524.2314.

### Example 8

### Synthesis of compound 8, (R)-2-amino-1-((R)-3-(4-amino-(4-phenoxyphenyl)-1H-pyrazolo[3,4-d]pyrimidin-1-yl)piperidin-1-yl)-3-(1H-indol-3-yl) propan-1-one

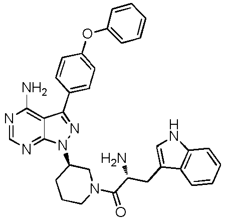

The synthesis of compound 8 was completed in the similar manner as described in example 1. Exact Mass (calculated value): 572.26; MS(ESI) m/z(M+1)⁺: 573.2615.

### Example 9

### Synthesis of compound 9, (S)-2-amino-1-((R)-3-(4-amino-(4-phenoxyphenyl)-1H-pyrazolo[3,4-d]pyrimidin-1-yl)piperidin-1-yl)-3-(1H-indol-3-yl)propan-1-one

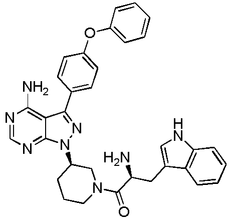

The synthesis of compound 9 was completed in the similar manner as described in example 1. Exact Mass (calculated value): 572.26; MS(ESI) m/z(M+1)⁺: 573.2614.

### Example 10

### Synthesis of compound 10, (R)-2-amino-1-((R)-3-(4-amino-(4-phenoxyphenyl)-1H-pyrazolo[3,4-d]pyrimidin-1-yl)piperidin-1-yl)-3-(4-hydroxyphenyl)propan-1-one

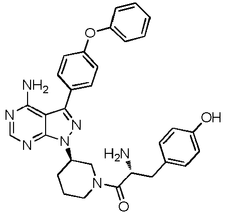

The synthesis of compound 10 was completed in the similar manner as described in example 1. Exact Mass (calculated value): 549.25; MS(ESI) m/z(M+1)⁺: 550.2514.

### Example 11

### Synthesis of compound 11, (S)-2-amino-1-((R)-3-(4-amino-(4-phenoxyphenyl)-1H-pyrazolo[3,4-d]pyrimidin-1-yl)piperidin-1-yl)-3-(4-hydroxyphenyl)propan-1-one

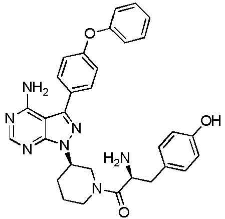

The synthesis of compound 11 was completed in the similar manner as described in example 1. Exact Mass (calculated value): 549.25; MS(ESI) m/z(M+1)⁺: 550.2516.

### Example 12

### Synthesis of compound 12, (S)-2-amino-1-((R)-3-(4-amino-(4-phenoxyphenyl)-1H-pyrazolo[3,4-d]pyrimidin-1-yl)piperidin-1-yl)-4-methylpentan-1-one

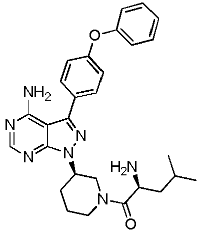

The synthesis of compound 12 was completed in the similar manner as described in example 1. Exact Mass (calculated value): 499.27; MS(ESI) m/z(M+1)⁺: 500.2716.

### Example 13

### Synthesis of compound 13, (R)-(3-(4-amino-(4-phenoxyphenyl)-1H-pyrazolo [3,4-d]pyrimidin-1-yl)piperidin-1-yl)(pyridin-3-yl)methanone

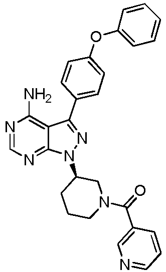

The synthesis of compound 13 in the similar manner as described in example 1. Exact Mass (calculated value): 491.21; MS(ESI) m/z(M+1)⁺: 492.2121.

### Example 14

### Synthesis of compound 14, (R)-(3-(4-amino-(4-phenoxyphenyl)-1H-pyrazolo [3,4-d]pyrimidin-1-yl)piperidin-1-yl)(piperazin-1-yl)methanone

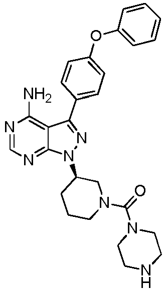

The synthesis of compound 14 was completed in the similar manner as described in example 1. Exact Mass (calculated value): 498.58; MS(ESI) m/z(M+1)⁺: 499.5821.

### Example 15

### Synthesis of compound 15, (R)-(3-(4-amino-(4-phenoxyphenyl)-1H-pyrazolo [3,4-d]pyrimidin-1-yl)piperidin-1-yl)(pyrimidin-5-yl)methanone

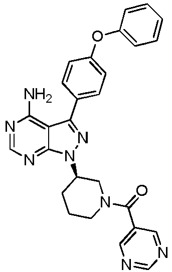

The synthesis of compound 15 was completed in the similar manner as described in example 1. Exact Mass (calculated value): 492.20; MS(ESI) m/z(M+1)⁺: 493.2021.

### Example 16

### Synthesis of compound 16, methyl (R)-4-(3-(4-amino-(4-phenoxyphenyl)-1H-pyrazolo[3,4-d]pyrimidin-1-yl)piperidin-1-carbonyl)benzoate

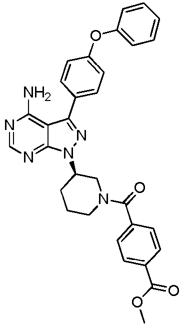

The synthesis of compound 16 was completed in the similar manner as described in example 1. Exact Mass (calculated value): 548.22; MS(ESI) m/z(M+1)⁺: 549.2221.

### Example 17

### Synthesis of compound 17, (R)-(3-(4-amino-(4-phenoxyphenyl)-1H-pyrazolo [3,4-d]pyrimidin-1-yl)piperidin-1-yl)(6-aminopyridin-3-yl)methanone

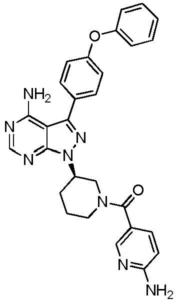

The synthesis of compound 17 was completed in the similar manner as described in example 1. Exact Mass (calculated value): 506.22; MS(ESI) m/z(M+1)⁺: 507.2221.

### Example 18

### Synthesis of compound 18, (R)-(3-(4-amino-(4-phenoxyphenyl)-1H-pyrazolo [3,4-d]pyrimidin-1-yl)piperidin-1-yl)-benzo[1,3]dioxol-5-yl-methanone

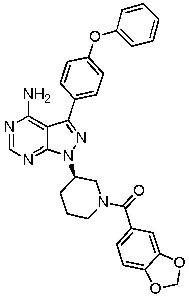

The synthesis of compound 18 was completed in the similar manner as described in example 1. Exact Mass (calculated value): 534.20; MS(ESI) m/z(M+1)⁺: 535.2021.

### Example 19

### Synthesis of compound 19, (R)-(3-(4-amino-(4-phenoxyphenyl)-1H-pyrazolo [3,4-d]pyrimidin-1-yl)piperidin-1-yl)-piperidin-4-yl-methanone

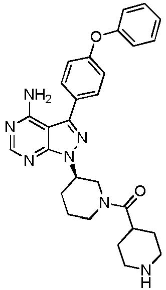

The synthesis of compound 19 was completed in the similar manner as described in example 1. Exact Mass (calculated value): 497.25; MS(ESI) m/z(M+1)⁺: 498.2521.

### Example 20

### Synthesis of compound 20, (R)-3-(3-(4-amino-(4-phenoxyphenyl)-1H-pyrazolo [3,4-d]pyrimidin-1-yl)piperidin-1-yl)-3-oxo-propionitrile

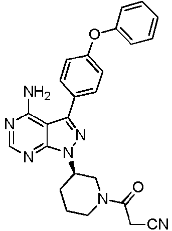

The synthesis of compound 20 was completed in the similar manner as described in example 1. Exact Mass (calculated value): 453.19; MS(ESI) m/z(M+1)⁺: 454.1922.

### Example 21

### Synthesis of compound 21, (R)-(3-(4-amino-(4-phenoxyphenyl)-1H-pyrazolo [3,4-d]pyrimidin-1-yl)piperidin-1-yl)(2-aminopyrimidin-5-yl)methanone

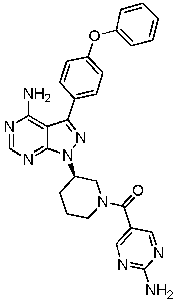

The synthesis of compound 21 was completed in the similar manner as described in example 1. Exact Mass (calculated value): 507.21; MS(ESI) m/z(M+1)⁺: 508.2134.

### Example 22

### Synthesis of compound 22, (R)-1-(3-(4-amino-(4-phenoxyphenyl)-1H-pyrazolo [3,4-d]pyrimidin-1-yl)piperidin-1-yl)-2-(dimethylamino)ethanone

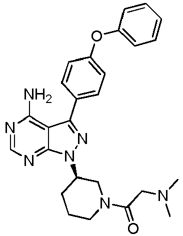

The synthesis of compound 22 was completed in the similar manner as described in example 1. Exact Mass (calculated value): 471.24; MS(ESI) m/z(M+1)⁺: 472.2431.

### Example 23

### Synthesis of compound 23, 1-((R)-3-(4-amino-(4-phenoxyphenyl)-1H-pyrazolo [3,4-d]pyrimidin-1-yl)piperidin-1-yl)-2-hydroxypropan-1-one

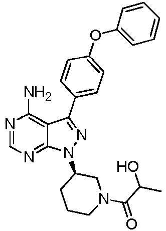

The synthesis of compound 23 was completed in the similar manner as described in example 1. Exact Mass (calculated value): 458.21; MS(ESI) m/z(M+1)⁺: 458.2124.

### Example 24

### Synthesis of compound 24, (R)-2-amino-1-((R)-3-(4-amino-(4-phenoxyphenyl)-1H-pyrazolo[3,4-d]pyrimidin-1-yl)piperidin-1-yl)propan-1-one

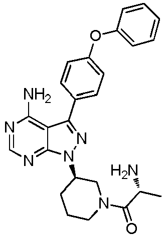

The synthesis of compound 24 was completed in the similar manner as described in example 1. Exact Mass (calculated value): 457.22; MS(ESI) m/z(M+1)⁺: 458.2234.

### Example 25

### Synthesis of compound 25, (2R,3R)-2-amino-1-((R)-3-(4-amino-(4-phenoxyphenyl) -1H-pyrazolo[3,4-d]pyrimidin-1-yl) piperidin-1-yl)-3- methylpentan-1-one

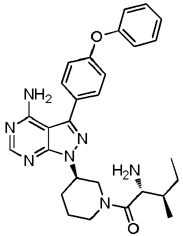

The synthesis of compound 25 was completed in the similar manner as described in example 1. Exact Mass (calculated value): 499.27; MS(ESI) m/z(M+1)⁺: 500.2731.

### Example 26

### Synthesis of compound 26, (R)-2-amino-1-((R)-3-(4-amino-(4-phenoxyphenyl)-1H-pyrazolo[3,4-d]pyrimidin-1-yl) piperidin-1-yl)-3- phenylpropan-1-one

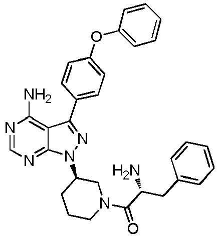

The synthesis of compound 26 was completed in the similar manner as described in example 1. Exact Mass (calculated value): 533.25; MS(ESI) m/z(M+1)⁺: 534.2541.

### Example 27

### Synthesis of compound 27, (R)-2-amino-1-((R)-3-(4-amino-(4-phenoxyphenyl)-1H -pyrazolo[3,4-d]pyrimidin-1-yl) piperidin-1-yl)-4- methylpentan-1-one

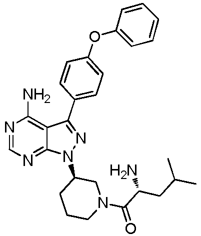

The synthesis of compound 27 was completed in the similar manner as described in example 1. Exact Mass (calculated value): 499.27; MS(ESI) m/z(M+1)⁺: 500.2733.

### Example 28

### Synthesis of compound 28, (R)-2-amino-1-((R)-3-(4-amino-(4-phenoxyphenyl)-1H -pyrazolo[3,4-d]pyrimidin-1-yl) pyrrolidin-1-yl)-4- methylpentan-1-one

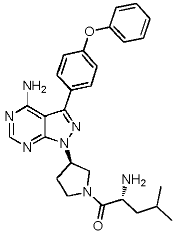

The synthesis of compound 28 was completed in the similar manner as described in example 1. Exact Mass (calculated value): 485.58; MS(ESI) m/z(M+1)⁺: 486.5833.

### Example 29

### Synthesis of compound 29, (R)-(3-(4-amino-(4-phenoxyphenyl)-1H-pyrazolo [3,4-d]pyrimidin-1-yl)piperidin-1-yl)(isoxazol-5-yl)methanone

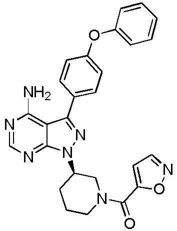

The synthesis of compound 29 was completed in the similar manner as described in example 1. Exact Mass (calculated value): 481.19; MS(ESI) m/z(M+1)⁺: 482.1921.

### Example 30

### Synthesis of compound 30, (R)-1-(1-methylsulfonyl)piperidin-3-yl)-3-(4-phenoxyphenyl)-1H-pyrazolo[3,4-d]pyrimidin-4-amine

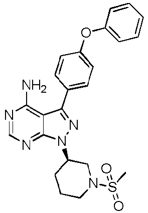

The synthesis of compound 30 was completed in the similar manner as described in example 1. Exact Mass (calculated value): 464.16; MS(ESI) m/z(M+1)⁺: 465.1622.

### Example 31

### Synthesis of compound 31, (R)-3-(4-amino-(4-phenoxyphenyl)-1H-pyrazolo [3,4-d]pyrimidin-1-yl)piperidin-1-yl)-2-morpholinyl ethanone

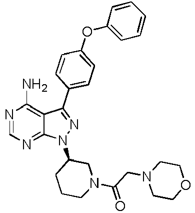

The synthesis of compound 31 was completed in the similar manner as described in example 1. Exact Mass (calculated value): 513.25; MS(ESI) m/z(M+1)⁺: 514.2531.

### Example 32

### Synthesis of compound 32, (R)-3-(4-amino-(4-phenoxyphenyl)-1H-pyrazolo [3,4-d]pyrimidin-1-yl)piperidin-1-yl)(cyclopentan-2-yl) ethanone

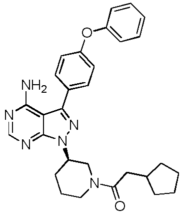

The synthesis of compound 32 was completed in the similar manner as described in example 1. Exact Mass (calculated value): 496.26; MS(ESI) m/z(M+1)⁺: 496.2631.

### Example 33

### Synthesis of compound 33, (R)-(3-(4-amino-(4-phenoxyphenyl)-1H-pyrazolo [3,4-d]pyrimidin-1-yl)piperidin-1-yl)(1-methylpiperidin-4-yl)methanone

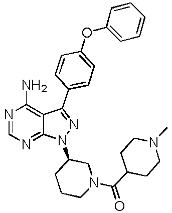

The synthesis of compound 33 was completed in the similar manner as described in example 1. Exact Mass (calculated value): 511.27; MS(ESI) m/z(M+1)⁺: 512.2731.

### Example 34

### Synthesis of compound 34, (R)-3-(4-amino-(4-phenoxyphenyl)-1H-pyrazolo [3,4-d]pyrimidin-1-yl)piperidin-1-yl)ethanone

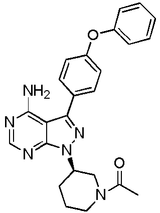

The synthesis of compound 34 was completed in the similar manner as described in example 1. Exact Mass (calculated value): 428.20; MS(ESI) m/z(M+1)⁺: 429.2031.

### Example 35

### Synthesis of compound 35, (R)-(3-(4-amino-(4-phenoxyphenyl)-1H-pyrazolo [3,4-d]pyrimidin-1-yl)piperidin-1-yl)(4-((4-methylpiperazin-1-yl)methyl)phenyl)methoa none

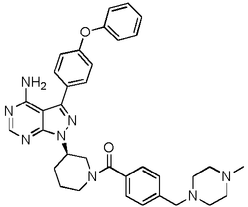

The synthesis of compound 35 was completed in the similar manner as described in example 1. Exact Mass (calculated value): 602.31; MS(ESI) m/z(M+1)⁺: 602.3123.

### Example 36

### Synthesis of compound 36, (R)-3-(4-amino-(4-phenoxyphenyl)-1H-pyrazolo [3,4-d]pyrimidin-1-yl)piperidin-1-yl)-3-chloroacetone

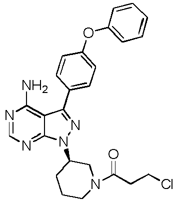

The synthesis of compound 36 was completed in the similar manner as described in example 1. Exact Mass (calculated value): 476.17; MS(ESI) m/z(M+1)⁺: 477.1723.

### Example 37

### Synthesis of compound 37, (R)-3-(4-amino-(4-phenoxyphenyl)-1H-pyrazolo [3,4-d]pyrimidin-1-yl)piperidin-1-yl)-2-bromoacetone

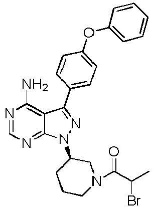

The synthesis of compound 37 was completed in the similar manner as described in example 1. Exact Mass (calculated value): 520.12; MS(ESI) m/z(M+1)⁺: 522.1217.

### Example 38

### Synthesis of compound 38, (R)-3-(4-amino-(4-phenoxyphenyl)-1H-pyrazolo [3,4-d]pyrimidin-1-yl)piperidin-1-yl)-2-chloroacetone

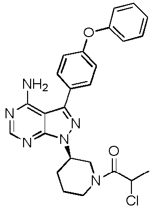

The synthesis of compound 38 was completed in the similar manner as described in example 1. Exact Mass (calculated value): 476.17; MS(ESI) m/z(M+1)⁺: 477.1725.

### Example 39

### Synthesis of compound 39, (R)-3-(4-amino-(4-phenoxyphenyl)-1H-pyrazolo [3,4-d]pyrimidin-1-yl)piperidin-1-yl)-2-(4-methylpiperazin-1-yl)ethanone

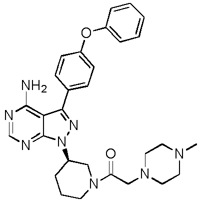

The synthesis of compound 39 was completed in the similar manner as described in example 1. Exact Mass (calculated value): 526.28; MS(ESI) m/z(M+1)⁺: 527.2826.

### Example 40

### Synthesis of compound 40, (R)-3-(4-phenoxyphenyl)-1-(1-(propylsulfonyl) piperidin-3-yl)-1H-pyrazolo[3,4-d]pyrimidin-4-amine

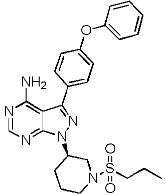

The synthesis of compound 40 was completed in the similar manner as described in example 1. Exact Mass (calculated value): 492.19; MS(ESI) m/z(M+1)⁺: 493.1921.

### Example 41

### Synthesis of compound 41 (R)-3-(4-phenoxyphenyl)-1-(1-(ethylsulfonyl)piperidin -3-yl)-1H-pyrazolo[3,4-d]pyrimidin-4-amine

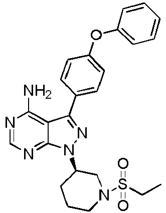

The synthesis of compound 41 was completed in the similar manner as described in example 1. Exact Mass (calculated value): 478.18; MS(ESI) m/z(M+1)⁺: 479.1821.

### Example 42

### Synthesis of compound 42 (R)-3-(4-amino-(4-phenoxyphenyl)-1H-pyrazolo [3,4-d]pyrimidin-1-yl)piperidin-1-yl)trichloroethanone

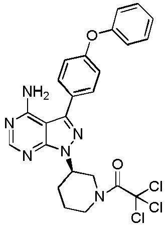

The synthesis of compound 42 was completed in the similar manner as described in example 1. Exact Mass (calculated value): 478.18; MS(ESI) m/z(M+1)⁺: 479.1821.

### Example 43

### Synthesis of compound 43, (R)-2-amino-1-(3-(4-amino-(4-phenoxyphenyl)-1H-pyrazolo[3,4-d]pyrimidin-1-yl)piperidin-1-yl) ethanone

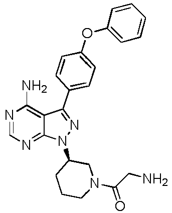

The synthesis of compound 43 was completed in the similar manner as described in example 1. Exact Mass (calculated value): 443.21; MS(ESI) m/z(M+1)⁺: 444.2118.

### Example 44

### Synthesis of compound 44, (R)-2-amino-1-((R)-3-(4-amino-(4-phenoxyphenyl)-1H -pyrazolo[3,4-d]pyrimidin-1-yl)piperidin-1-yl)-3-hydroxybutanone

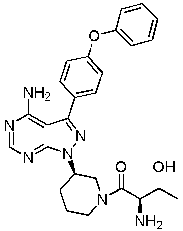

The synthesis of compound 44 was completed in the similar manner as described in example 1. Exact Mass (calculated value): 487.23; MS(ESI) m/z(M+1)⁺: 488.2321.

### Example 45

### Synthesis of compound 45, (R)-2-amino-1-((R)3-(4-amino-(4-phenoxyphenyl)-1H-pyrazolo[3,4-d]pyrimidin-1-yl)piperidin-1-yl)-3-methylbutanone

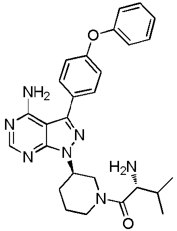

The synthesis of compound 45 was completed in the similar manner as described in example 1. Exact Mass (calculated value): 485.25; MS(ESI) m/z(M+1)⁺: 486.2531.

### Example 46

### Synthesis of compound 46, (R)-3-amino-1-(3-(4-amino-(4-phenoxyphenyl)-1H-pyrazolo[3,4-d]pyrimidin-1-yl)piperidin-1-yl)acetone

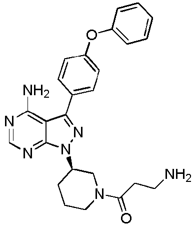

The synthesis of compound 46 was completed in the similar manner as described in example 1. Exact Mass (calculated value): 457.22; MS(ESI) m/z(M+1)⁺: 457.2221.

### Example 47

### Synthesis of compound 47, (R)-2-amino-1-((R)-(3-(4-amino-(4-phenoxyphenyl)-1H-pyrazolo[3,4-d]pyrimidin-1-yl)piperidin-1-yl)acetone

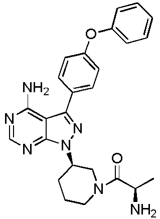

The synthesis of compound 47 was completed in the similar manner as described in example 1. Exact Mass (calculated value): 457.22; MS(ESI) m/z(M+1)⁺: 458.2226.

### Example 48

### Synthesis of compound 48, (R)-2-amino-1-((R)-(3-(4-amino-(4-phenoxyphenyl)-1H-pyrazolo[3,4-d]pyrimidin-1-yl)piperidin-1-yl)-3-hydroxyacetone

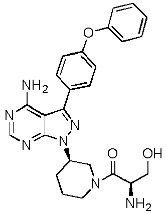

The synthesis of compound 48 was completed in the similar manner as described in example 1. Exact Mass (calculated value): 473.22; MS(ESI) m/z(M+1)⁺: 474.2225.

### Example 49

### Synthesis of compound 49, (R)-3-(4-amino-(4-phenoxyphenyl)-1H-pyrazolo[3,4-d] pyrimidin-1-yl)piperidin-1-yl)-3,3-dimethyl butanone

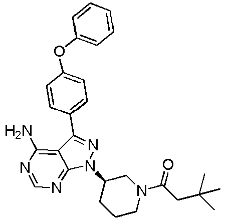

The synthesis of compound 49 was completed in the similar manner as described in example 1. Exact Mass (calculated value): 473.22; MS(ESI) m/z(M+1)⁺: 474.2225.

### Example 50

### Synthesis of compound 50, (R)-3-(4-amino-(4-phenoxyphenyl)-1H-pyrazolo[3,4-d] pyrimidin-1-yl)piperidin-1-yl)-2,2-dimethyl butanone

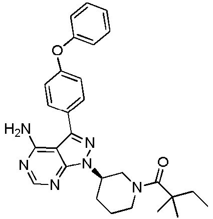

The synthesis of compound 50 was completed in the similar manner as described in example 1. Exact Mass (calculated value): 473.22; MS(ESI) m/z(M+1)⁺: 474.2226.

### Example 51

### Synthesis of compound 51, (R)-3-(4-amino-(4-phenoxyphenyl)-1H-pyrazolo[3,4-d] pyrimidin-1-yl)piperidin-1-yl)-2,2,3-trifluoroacetone

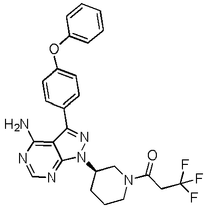

The synthesis of compound 51 was completed in the similar manner as described in example 1. Exact Mass (calculated value): 496.18; MS(ESI) m/z(M+1)⁺: 497.1824.

### Example 52

### Synthesis of compound 52, (R)-3-(4-amino-(4-phenoxyphenyl)-1H-pyrazolo[3,4-d] pyrimidin-1-yl)piperidin-1-yl)-2-cyclopropyl ethanone

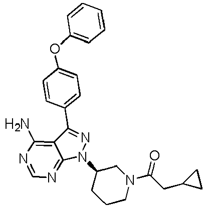

The synthesis of compound 52 was completed in the similar manner as described in example 1. Exact Mass (calculated value): 468.23; MS(ESI) m/z(M+1)⁺: 469.2327.

### Example 53

### Synthesis of compound 53, (R)-3-(4-amino-(4-phenoxyphenyl)-1H-pyrazolo[3,4-d] pyrimidin-1-yl)piperidin-1-yl)-3-methyl butanone

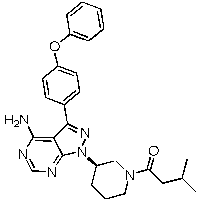

The synthesis of compound 53 was completed in the similar manner as described in example 1. Exact Mass (calculated value): 470.24; MS(ESI) m/z(M+1)⁺: 471.2427.

### Example 54

### Synthesis of compound 54, (R)-3-(4-amino-(4-phenoxyphenyl)-1H-pyrazolo[3,4-d] pyrimidin-1-yl)piperidin-1-yl) butanone

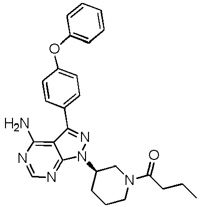

The synthesis of compound 54 was completed in the similar manner as described in example 1. Exact Mass (calculated value): 456.23; MS(ESI) m/z(M+1)⁺: 457.2327.

### Example 55

### Synthesis of compound 55, (R)-3-(4-amino-(4-phenoxyphenyl)-1H-pyrazolo[3,4-d] pyrimidin-1-yl)piperidin-1-yl)-2,2-dimethylacetone

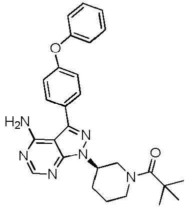

The synthesis of compound 55 was completed in the similar manner as described in example 1. Exact Mass (calculated value): 470.24; MS(ESI) m/z(M+1)⁺: 471.2426.

### Example 56

### Synthesis of compound 56, (S)-4-amino-5-((R)-3-(4-amino-(4-phenoxyphenyl)-1H -pyrazolo[3,4-d]pyrimidin-1-yl)piperidin-1-yl)-5- oxo-pentanamide

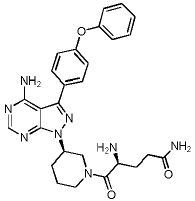

The synthesis of compound 56 was completed in the similar manner as described in example 1. Exact Mass (calculated value): 514.24; MS(ESI) m/z(M+1)⁺: 515.2428.

### Example 57

### Synthesis of compound 57, (R)-4-amino-5-((R)-3-(4-amino-(4-phenoxyphenyl)-1H -pyrazolo[3,4-d]pyrimidin-1-yl)piperidin-1-yl)-5-oxo-pentanamide

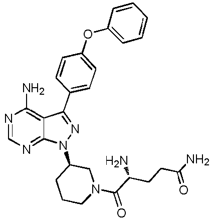

The synthesis of compound 57 was completed in the similar manner as described in example 1. Exact Mass (calculated value): 514.24; MS(ESI) m/z(M+1)⁺: 515.2427.

### Example 58

### Synthesis of compound 58, (R)-1-(3-(4-amino-(4-phenoxyphenyl)-1H-pyrazolo [3,4-d]pyrimidin-1-yl)piperidin-1-yl)-3-(4-methylpiperazin-1-yl) acetone

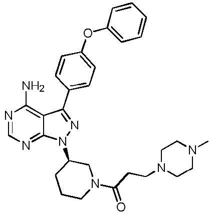

The synthesis of compound 58 was completed in the similar manner as described in example 1. Exact Mass (calculated value): 540.31; MS(ESI) m/z(M+1)⁺: 541.3135.

### Example 59

### Synthesis of compound 59, tert-butyl (R)-(2-(3-(4-amino-3-(4-phenoxyphenyl)-1H -pyrazolo[3,4-d]pyrimidin-1-yl)piperidin-1-yl)-2-carbonyl)ethyl carbamate

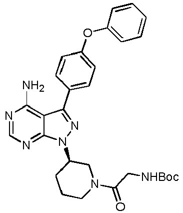

The synthesis of compound 59 was completed in the similar manner as described in example 1. Exact Mass (calculated value): 543.26; MS(ESI) m/z(M+1)⁺: 544.2627.

### Example 60

### Synthesis of compound 60, methyl (R)-4-(3-(4-amino-(4-phenoxyphenyl)-1H-pyrazolo[3,4-d]pyrimidin-1-yl)piperidin-1-carbonyl)piperidin-1- formate

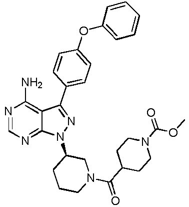

The synthesis of compound 60 was completed in the similar manner as described in example 1. Exact Mass (calculated value): 555.26; MS(ESI) m/z(M+1)⁺: 556.2627.

### Example 61

### Synthesis of compound 61, (R)-1-((R)-3-(4-amino-(4-phenoxyphenyl)-1H-pyrazolo[3,4-d]pyrimidin-1-yl)piperidin-1-yl)-2-hydroxyl-4- methylpentan-1-one

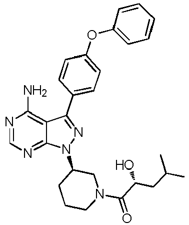

The synthesis of compound 61 was completed in the similar manner as described in example 1. Exact Mass (calculated value): 500.25; MS(ESI) m/z(M+1)⁺: 501.2527.

### Example 62

### Synthesis of compound 62, (R)-3-(4-amino-(4-phenoxyphenyl)-1H-pyrazolo[3,4-d] pyrimidin-1-yl)piperidin-1-yl)-5-aminopentanone

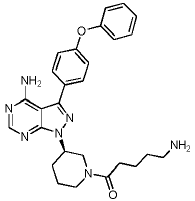

The synthesis of compound 62 was completed in the similar manner as described in example 1. Exact Mass (calculated value): 485.25; MS(ESI) m/z(M+1)⁺: 486.2526.

### Example 63

### Synthesis of compound 63, (R)-3-(4-amino-(4-phenoxyphenyl)-1H-pyrazolo[3,4-d] pyrimidin-1-yl)piperidin-1-yl)-2-hydroxyhexan-1-one

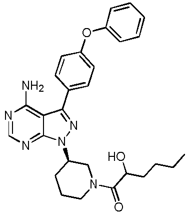

The synthesis of compound 63 was completed in the similar manner as described in example 1. Exact Mass (calculated value): 500.25; MS(ESI) m/z(M+1)⁺: 501.2528.

### Example 64

### Synthesis of compound 64, (R)-3-(4-amino-(4-phenoxyphenyl)-1H-pyrazolo[3,4-d] pyrimidin-1-yl)pyrrolidin-1-yl)-2-aminoethanone

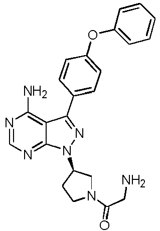

The synthesis of compound 64 was completed in the similar manner as described in example 1. Exact Mass (calculated value): 429.19; MS(ESI) m/z(M+1)⁺: 430.1928.

### Example 65

### Synthesis of compound 65, (R)-3-(4-amino-(4-phenoxyphenyl)-1H-pyrazolo[3,4-d] pyrimidin-1-yl)pyrrolidin-1-yl)-2-(dimethylamino)ethanone

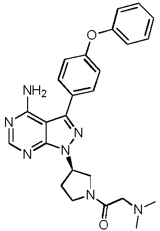

The synthesis of compound 65 was completed in the similar manner as described in example 1. Exact Mass (calculated value): 457.22; MS(ESI) m/z(M+1)⁺: 458.2225.

### Example 66

### Synthesis of compound 66, tert-butyl (R)-(2-(3-(4-amino-(4-phenoxyphenyl)-1H-pyrazolo[3,4-d]pyrimidin-1-yl)pyrrolidin-1-yl)-2-carbonyl) ethylcarbamate

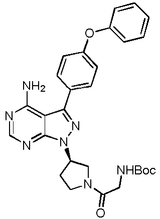

The synthesis of compound 66 was completed in the similar manner as described in example 1. Exact Mass (calculated value): 529.24; MS(ESI) m/z(M+1)⁺: 530.2427.

### Example 67

### Synthesis of compound 67, (R)-1-(1-(methylsulfonyl)pyrrolidin-3-yl)-3-(4-phenoxyphenyl)-1H-pyrazolo[3,4-d]pyrimidin-4-amine

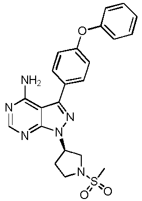

The synthesis of compound 67 was completed in the similar manner as described in example 1. Exact Mass (calculated value): 450.15; MS(ESI) m/z(M+1)⁺: 451.1527

### Example 68

### Synthesis of compound 68, (4-(4-amino-(4-phenoxyphenyl)-1H-pyrazolo[3,4-d] pyrimidin-1-yl)piperidin-1-yl)(1-methylpiperidin-4-yl) methanone

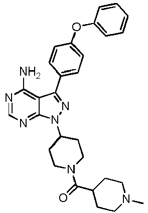

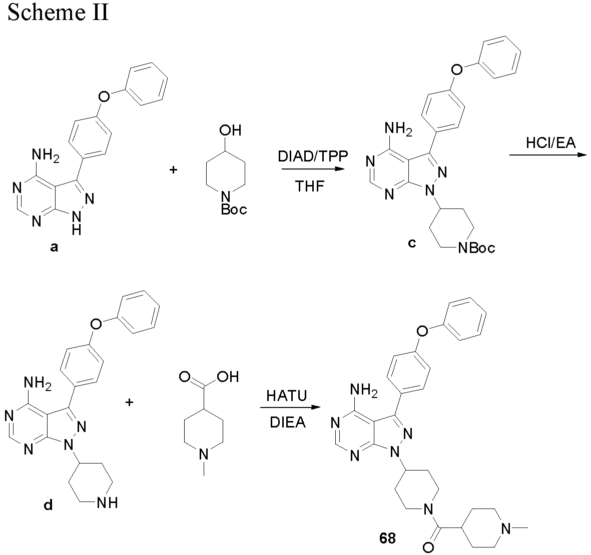

As shown in scheme II, the synthesis of compound 68 used a method similar to that described in example 1, wherein a was used to synthesize c, but replacing the raw material from (S)-N-tert-butoxycarbonyl-3-piperidinol to N-tert-butoxycarbonyl-4-piperidinol. Compound c was dissolved in dichlormethane, added with trifluoroacetic acid, stirred for 2 h at room temperature, and then concentrated. The residue was added to ethyl acetate and aqueous potassium carbonate, and then the organic phase was dried, filtered, and concentrated. The resultant was added with a solution of 4.0 M HCl in 1,4-dioxane with stirring, and the solid was then collected and washed with ethyl acetate. The solid was then dissolved in ethyl acetate and a solution of potassium carbonate, and the organic phase was dried and concentrated to obtain intermediate d. The compound d (20 mg, 0.05 mmol) was dissolved in N,N-dimethylformamide (2 ml), and then 1-methylpiperidin-4-carboxylic acid (7.4 mg, 0.05 mmol), diisopropylethylamine (7.4mg, 0.05 mmol), and HATU (21.6mg, 0.05mmol) were added. After stirring for 0.5 h at room temperature, the mixture was diluted with ethyl acetate, washed with water, dried, and concentrated by column chromatography to yield 12 mg of white solid compound 68. Exact Mass (calculated value):511.62; MS(ESI) m/z(M+1)⁺: 512.6227.

### Example 69

### Synthesis of compound 69, 1-(4-(4-amino-(4-phenoxyphenyl)-1H-pyrazolo[3,4-d] pyrimidin-1-yl)piperidin-1-yl)-2-(4-methylpiperazin-1-yl)ethanone

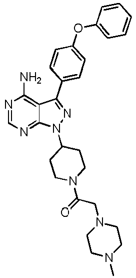

The synthesis of compound 69 was completed in the similar manner as described in example 68. Exact Mass (calculated value): 526.63; MS(ESI) m/z(M+1)⁺: 527.6329.

### Example 70

### Synthesis of compound 70, 1-(4-(4-amino-(4-phenoxyphenyl)-1H-pyrazolo[3,4-d] pyrimidin-1-yl)piperidin-1-yl)-2- morpholinyl-ethanone

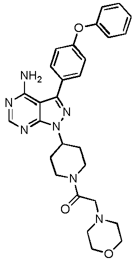

The synthesis of compound 70 was completed in the similar manner as described in example 68. Exact Mass (calculated value): 513.59; MS(ESI) m/z(M+1)⁺: 514.5917.

### Example 71

### Synthesis of compound 71, (4-(4-amino-(4-phenoxyphenyl)-1H-pyrazolo[3,4-d] pyrimidin-1-yl)piperidin-1-yl)-(4-((4-methylpiperazin-1-yl)methyl)phenyl)methanone

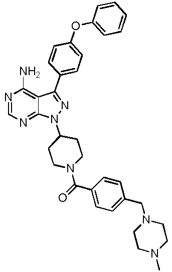

The synthesis of compound 71 was completed in the similar manner as described in example 68. Exact Mass (calculated value): 602.73; MS(ESI) m/z(M+1)⁺: 603.7325.

### Example 72

### Synthesis of compound 72, 1-(4-(4-amino-(4-phenoxyphenyl)-1H-pyrazolo[3,4-d] pyrimidin-1-yl)piperidin-1-yl)-2-(dimethylamino)ethanone

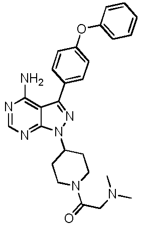

The synthesis of compound 72 was completed in the similar manner as described in example 68. Exact Mass (calculated value): 471.55; MS(ESI) m/z(M+1)⁺: 472.5532.

### Example 73

### Synthesis of compound 73, 1-(1-(methylsulfonyl)piperidin-4-yl)-3-(4-phenoxyphenyl)-1H-pyrazolo[3,4-d]pyrimidin-4-amine

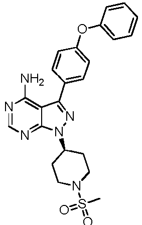

The synthesis of compound 73 was completed in the similar manner as described in example 68. Exact Mass (calculated value): 464.54; MS(ESI) m/z(M+1)⁺: 465.5423.

### Example 74

### Synthesis of compound 74, (R)-2-amino-1-(4-(4-amino-(4-phenoxyphenyl)-1H-pyrazolo[3,4-d]pyrimidin-1-yl)piperidin-1-yl)-4-methylpentan-1-one

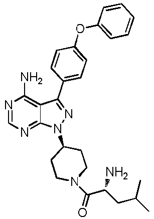

The synthesis of compound 74 was completed in the similar manner as described in example 68. Exact Mass (calculated value): 499.61; MS(ESI) m/z(M+1)⁺: 500.6128.

### Example 75

### Synthesis of compound 75, 1-(4-(4-amino-(4-phenoxyphenyl)-1H-pyrazolo[3,4-d] pyrimidin-1-yl)piperidin-1-yl)-2-(methylamino)ethanone

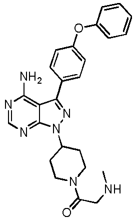

The synthesis of compound 75 was completed in the similar manner as described in example 68. Exact Mass (calculated value): 457.53; MS(ESI) m/z(M+1)⁺: 458.5316.

### Example 76

### Synthesis of compound 76, N-(4-(4-amino-(4-phenoxyphenyl)-1H-pyrazolo[3,4-d] pyrimidin-1-yl)cyclohexyl)-2-(dimethylamino)acetamide

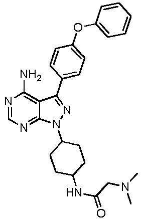

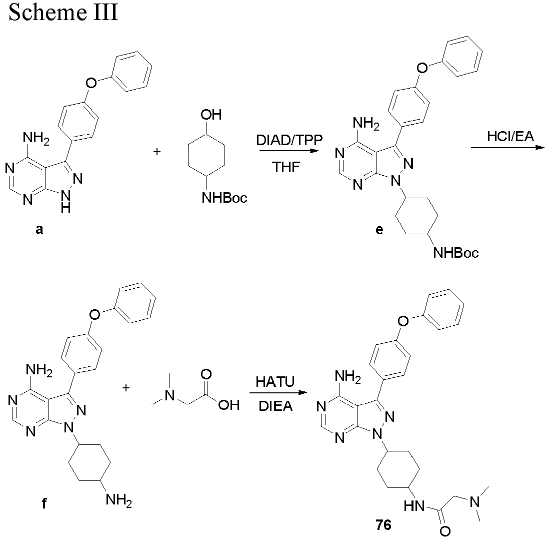

As shown in scheme III, the synthesis of compound 76 used a method similar to that described in example 1, wherein a was used to synthesize e, but replacing the raw material from (S)-N-tert-butoxycarbonyl-3-piperidinol to N-Boc-4-aminocyclohexanol. The compound e was dissolved in dichlormethane, and then the solution was added with trifluoroacetic acid, stirred for 2 h at room temperature, and concentrated. The residue was added to ethyl acetate and aqueous potassium carbonate, and then the organic phase was dried, filtered, concentrated. The resultant was added with a solution of 4.0 M HCl in 1,4-dioxane with stirring, and the solid was then collected and washed with ethyl acetate. The solid was then dissolved in ethyl acetate and a solution of potassium carbonate, and the organic phase was dried and concentrated to obtain intermediate f. The compound f (20 mg, 0.05 mmol) was dissolved in N,N-dimethylformamide (2 ml), and then 2-(dimethylamino)acetic acid (5.1 mg, 0.05 mmol), diisopropylethylamine (7.4 mg, 0.05 mmol), and HATU (21.6 mg, 0.05 mmol) were added. After stirring for 0.5 h at room temperature, the mixture was diluted with ethyl acetate, washed with water, dried, concentrated by column chromatography to yield 16 mg of white solid compound 76. Exact Mass (calculated value): 485.58; MS(ESI) m/z(M+1)⁺: 486.5816.

### Example 77

### Synthesis of compound 77, (R)-2-amino-N-(4-(4-amino-3-(4-phenoxyphenyl)-1H-pyrazolo[3,4-d]pyrimidin-1-yl)cyclohexyl)-4-methyl-pentanamide hydrochloride

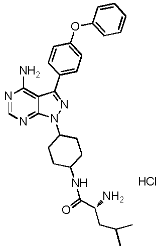

The synthesis of compound 77 was completed in the similar manner as described in example 76. Exact Mass (calculated value): 513.64; MS(ESI) m/z(M+1)⁺: 514.6423.

### Example 78

### Synthesis of compound 78, N-(4-(4-amino-3-(4-phenoxyphenyl)-1H-pyrazolo [3,4-d]pyrimidin-1-yl)cyclohexyl)methylsulfonamide

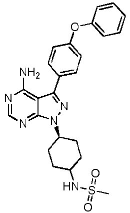

The synthesis of compound 78 was completed in the similar manner as described in example 76. Exact Mass (calculated value): 478.57; MS(ESI) m/z(M+1)⁺: 479.5717.

### Example 79

### Synthesis of compound 79, N-(4-(4-amino-3-(4-phenoxyphenyl)-1H-pyrazolo [3,4-d]pyrimidin-1-yl)cyclohexyl)-2-(methylamino)acetamide

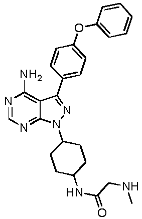

The synthesis of compound 79 was completed in the similar manner as described in example 76. Exact Mass (calculated value): 471.55; MS(ESI) m/z(M+1)⁺: 472.5521.

### Example 80

### Synthesis of compound 80, N-(4-(4-amino-3-(4-phenoxyphenyl)-1H-pyrazolo [3,4-d]pyrimidin-1-yl)cyclohexyl)-1-methylaminopiperidin-4-formamide

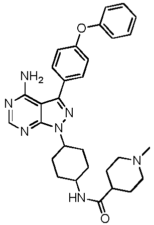

The synthesis of compound 80 was completed in the similar manner as described in example 76. Exact Mass (calculated value): 471.55; MS(ESI) m/z(M+1)⁺: 472.5521.

### Example 81

### Synthesis of compound 81, N-(4-(4-amino-3-(4-phenoxyphenyl)-1H-pyrazolo [3,4-d]pyrimidin-1-yl)cyclohexyl)-2-(4-methylpiperazin-1-yl)acetamide

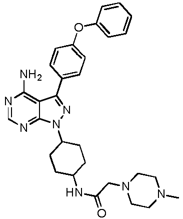

The synthesis of compound 81 was completed in the similar manner as described in example 76. Exact Mass (calculated value): 540.66; MS(ESI) m/z(M+1)⁺: 541.6625.

### Example 82

### Synthesis of compound 82, N-(4-(4-amino-3-(4-phenoxyphenyl)-1H-pyrazolo [3,4-d]pyrimidin-1-yl)cyclohexyl)-2- morpholinyl-acetamide

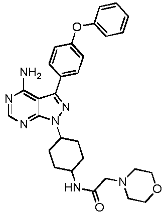

The synthesis of compound 82 was completed in the similar manner as described in example 76. Exact Mass (calculated value): 527.62; MS(ESI) m/z(M+1)⁺: 528.6223.

### Example 83

### Synthesis of compound 83, N-(4-(4-amino-3-(4-phenoxyphenyl)-1H-pyrazolo [3,4-d]pyrimidin-1-yl)cyclohexyl)-2-cyanoacetamide

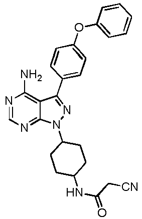

The synthesis of compound 83 was completed in the similar manner as described in example 76. Exact Mass (calculated value): 467.52; MS(ESI) m/z(M+1)⁺: 468.5218.

### Example 84

### Synthesis of compound 84, (R)-2-amino-N-(4-(4-amino-3-(4-phenoxyphenyl)-1H-pyrazolo[3,4-d]pyrimidin-1-yl)cis-1,4-cyclohexyl)-4-methyl-pentanamide hydrochloride

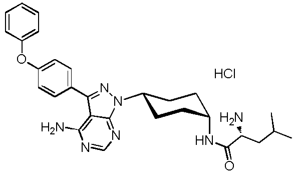

The synthesis of compound 84 was completed in the similar manner as described in example 76, except that trans-4-Boc-aminocyclohexanol was used in place of the raw material N-Boc-4-aminocyclohexanol. Exact Mass (calculated value): 513.64; MS(ESI) m/z(M+1)⁺: 514.6424.

### Example 85

### Synthesis of compound 85, (R)-2-amino-N-(4-(4-amino-3-(4-phenoxyphenyl)-1H-pyrazolo[3,4-d]pyrimidin-1-yl)trans-1,4-cyclohexyl)-4-methyl-pentanamide hydrochloride

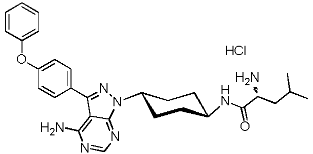

The synthesis of compound 85 was completed in the similar manner as described in example 76, except that cis-4-Boc-aminocyclohexanol was used in place of the raw material N-Boc-4-aminocyclohexanol. Exact Mass (calculated value): 513.64; MS(ESI) m/z(M+1)⁺: 514.6425.

### Example 86

### Synthesis of compound 86, (R)-2-amino-N-(4-(4-amino-(4-phenoxyphenyl)-1H-pyrazolo[3,4-d]pyrimidin-1-yl)cyclohexyl)-3-methyl butyramide hydrochloride

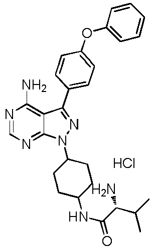

The synthesis of compound 86 was completed in the similar manner as described in example 76. Exact Mass (calculated value): 499.61; MS(ESI) m/z(M+1)⁺: 500.2785.

### Example 87

### Synthesis of compound 87, (R)-N-(4-(4-amino-(4-phenoxyphenyl)-1H-pyrazolo [3,4-d]pyrimidin-1-yl)cyclohexyl)-3-methyl-2-(methylamino)butyramide hydrochloride

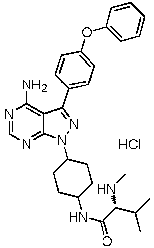

The synthesis of compound 87 was completed in the similar manner as described in example 76. Exact Mass (calculated value): 513.63; MS(ESI) m/z(M+1)⁺: 514.2985.

### Example 88

### Synthesis of compound 88, (R)-N-(4-(4-amino-(4-phenoxyphenyl)-1H-pyrazolo [3,4-d]pyrimidin-1-yl)cyclohexyl)-2-(dimethylamino)-3-methylbutyramide

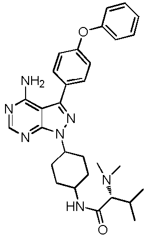

The synthesis of compound 88 was completed in the similar manner as described in example 76. Exact Mass (calculated value): 527.66; MS(ESI) m/z(M+1)⁺: 528.3065.

### Example 89

### Synthesis of compound 89, (S)-2-amino-N-(4-(4-amino-(4-phenoxyphenyl)-1H-pyrazolo[3,4-d]pyrimidin-1-yl)cyclohexyl)-3-methylbutyramide hydrochloride

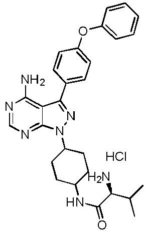

The synthesis of compound 89 was completed in the similar manner as described in example 76. Exact Mass (calculated value): 499.61; MS(ESI) m/z(M+1)+: 500.2787.

### Example 90

### Synthesis of 90, (S)-N-(4-(4-amino-(4-phenoxyphenyl)-1H-pyrazolo[3,4-d] pyrimidin-1-yl)cyclohexyl)-3-methyl-2-(methylamino)butyramide hydrochloride

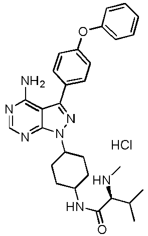

The synthesis of compound 90 was completed in the similar manner as described in example 76. Exact Mass (calculated value): 513.63; MS(ESI) m/z(M+1)⁺: 514.2986.

### Example 91

### Synthesis of 91, (S)-N-(4-(4-amino-(4-phenoxyphenyl)-1H-pyrazolo[3,4-d] pyrimidin-1-yl)cyclohexyl)- 2-(dimethylamino)-3-methylbutyramide

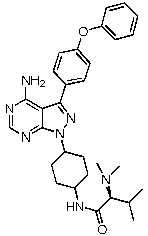

The synthesis of compound 91 was completed in the similar manner as described in example 76. Exact Mass (calculated value): 527.66; MS(ESI) m/z(M+1)⁺: 528.3067.

### Example 92

### Synthesis of 92, (R)-N-(4-(4-amino-(4-phenoxyphenyl)-1H-pyrazolo[3,4-d] pyrimidin-1-yl)cyclohexyl)- 4-methyl-2-(methylamino)pentanamide hydrochloride

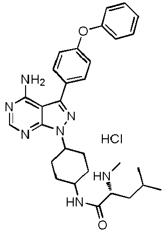

The synthesis of compound 92 was completed in the similar manner as described in example 76. Exact Mass (calculated value): 527.66; MS(ESI) m/z(M+1)⁺: 528.3063.

### Example 93

### Synthesis of 93, (R)-N-(4-(4-amino-(4-phenoxyphenyl)-1H-pyrazolo[3,4-d] pyrimidin-1-yl)cyclohexyl)-2-(dimethylamino)-4-methylpentanamide

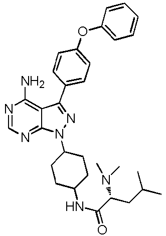

The synthesis of compound 93 was completed in the similar manner as described in example 76. Exact Mass (calculated value): 541.69; MS(ESI) m/z(M+1)⁺: 542.3263.

### Example 94

### Synthesis of 94, (S)-2-amino-N-(4-(4-amino-(4-phenoxyphenyl)-1H-pyrazolo [3,4-d]pyrimidin-1-yl)cyclohexyl)-4-methylpentanamide

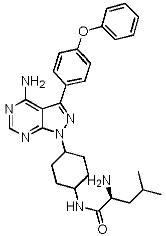

The synthesis of compound 94 was completed in the similar manner as described in example 76. Exact Mass (calculated value): 513.63; MS(ESI) m/z(M+1)⁺: 514.2963.

### Example 95

### Synthesis of 95, (S)-N-(4-(4-amino-(4-phenoxyphenyl)-1H-pyrazolo[3,4-d] pyrimidin-1-yl)cyclohexyl)-4-methyl-2-(methylamino)pentanamide hydrochloride

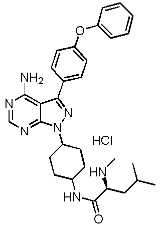

The synthesis of compound 95 was completed in the similar manner as described in example 76. Exact Mass (calculated value): 527.66; MS(ESI) m/z(M+1)⁺: 528.3065.

### Example 96

### Synthesis of 96, (S)-N-(4-(4-amino-(4-phenoxyphenyl)-1H-pyrazolo[3,4-d] pyrimidin-1-yl)cyclohexyl)- 2-(dimethylamino)-4-methylpentanamide

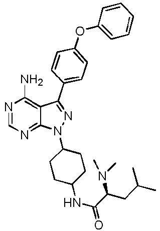

The synthesis of compound 96 was completed in the similar manner as described in example 76. Exact Mass (calculated value): 541.69; MS(ESI) m/z(M+1)⁺: 542.3266.

### Example 97

### Synthesis of 97, (R)-2-amino-N-(4-(4-amino-(4-phenoxyphenyl)-1H-pyrazolo [3,4-d]pyrimidin-1-yl)cyclohexyl)-butyramide hydrochloride

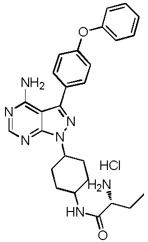

The synthesis of compound 97 was completed in the similar manner as described in example 76. Exact Mass (calculated value): 485.58; MS(ESI) m/z(M+1)⁺: 486.2666.

### Example 98

### Synthesis of 98, (R)-N-(4-(4-amino-(4-phenoxyphenyl)-1H-pyrazolo[3,4-d] pyrimidin-1-yl)cyclohexyl)-2-(methylamino)-butyramide hydrochloride

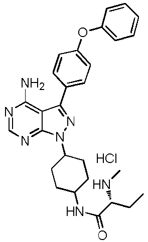

The synthesis of compound 98 was completed in the similar manner as described in example 76. Exact Mass (calculated value): 499.61; MS(ESI) m/z(M+1)⁺: 500.2766.

### Example 99

### Synthesis of compound 99, (R)-N-(4-(4-amino-(4-phenoxyphenyl)-1H-pyrazolo [3,4-d]pyrimidin-1-yl)cyclohexyl)-2-(dimethylamino)-butyramide

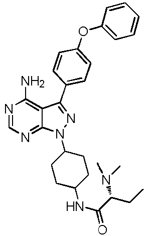

The synthesis of compound 99 was completed in the similar manner as described in example 76. Exact Mass (calculated value): 513.63; MS(ESI) m/z(M+1)⁺: 514.2966.

### Example 100

### Synthesis of compound 100, (S)-2-amino-N-(4-(4-amino-(4-phenoxyphenyl)-1H-pyrazolo[3,4-d]pyrimidin-1-yl)cyclohexyl)-butyramide hydrochloride

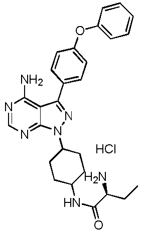

The synthesis of compound 100 was completed in the similar manner as described in example 76. Exact Mass (calculated value): 485.58; MS(ESI) m/z(M+1)⁺: 486.2669.

### Example 101

### Synthesis of compound 101, (S)-N-(4-(4-amino-(4-phenoxyphenyl)-1H-pyrazolo [3,4-d]pyrimidin-1-yl)cyclohexyl)-2-(methylamino)butyramide hydrochloride

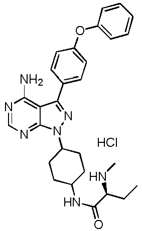

The synthesis of compound 101 was completed in the similar manner as described in example 76. Exact Mass (calculated value): 499.61; MS(ESI) m/z(M+1)⁺: 500.2769.

### Example 102

### Synthesis of compound 102, (S)-N-(4-(4-amino-(4-phenoxyphenyl)-1H-pyrazolo [3,4-d]pyrimidin-1-yl)cyclohexyl)-2-(dimethylamino)butyramide

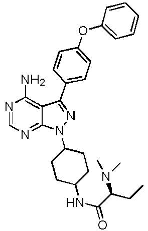

The synthesis of compound 102 was completed in the similar manner as described in example 76. Exact Mass (calculated value): 513.63; MS(ESI) m/z(M+1)⁺: 514.2963.

### Example 103

### Synthesis of compound 103, (R)-2-amino-N-(4-(4-amino-(4-phenoxyphenyl)-1H-pyrazolo[3,4-d]pyrimidin-1-yl)cyclohexyl)-pentanamide hydrochloride

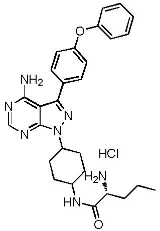

The synthesis of compound 103 was completed in the similar manner as described in example 76. Exact Mass (calculated value): 499.61; MS(ESI) m/z(M+1)⁺: 500.2763.

### Example 104

### Synthesis of compound 104, (R)-N-(4-(4-amino-(4-phenoxyphenyl)-1H-pyrazolo [3,4-d]pyrimidin-1-yl)cyclohexyl)-2-(methylamino)-pentanamide hydrochloride

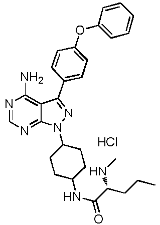

The synthesis of compound 104 was completed in the similar manner as described in example 76. Exact Mass (calculated value): 513.63; MS(ESI) m/z(M+1)⁺: 514.2963.

### Example 105

### Synthesis of compound 105, (R)-N-(4-(4-amino-(4-phenoxyphenyl)-1H-pyrazolo [3,4-d]pyrimidin-1-yl)cyclohexyl)-2-(dimethylamino)-pentanamide

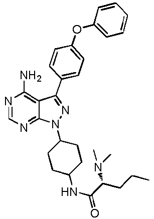

The synthesis of compound 105 was completed in the similar manner as described in example 76. Exact Mass (calculated value): 527.66; MS(ESI) m/z(M+1)⁺: 528.3063.

### Example 106

### Synthesis of compound 106, (S)-2-amino-N-(4-(4-amino-(4-phenoxyphenyl)-1H-pyrazolo[3,4-d]pyrimidin-1-yl)cyclohexyl)-pentanamide hydrochloride

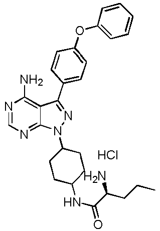

The synthesis of compound 106 was completed in the similar manner as described in example 76. Exact Mass (calculated value): 499.61; MS(ESI) m/z(M+1)⁺: 500.2769.

### Example 107

### Synthesis of 107, (S)-N-(4-(4-amino-(4-phenoxyphenyl)-1H-pyrazolo[3,4-d] pyrimidin-1-yl)cyclohexyl)-2-(methylamino)-pentanamide hydrochloride

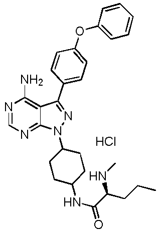

The synthesis of compound 107 was completed in the similar manner as described in example 76. Exact Mass (calculated value): 513.63; MS(ESI) m/z(M+1)⁺: 514.2971.

### Example 108

### Synthesis of 108, (S)-N-(4-(4-amino-(4-phenoxyphenyl)-1H-pyrazolo[3,4-d] pyrimidin-1-yl)cyclohexyl)-2-(dimethylamino)-pentanamide

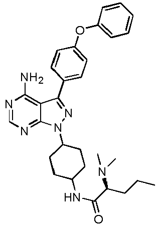

The synthesis of compound 108 was in the similar manner as described in example 76. Exact Mass (calculated value): 527.66; MS(ESI) m/z(M+1)⁺: 528.3068.

### Example 109

### Synthesis of compound 109, (R)-2-amino-N-(4-(4-amino-3-(4-phenoxyphenyl)-1H -pyrazolo[3,4-d]pyrimidin-1-yl)cis-1,4-cyclohexyl)-4-methyl-2-(methylamino)pentana mide hydrochloride

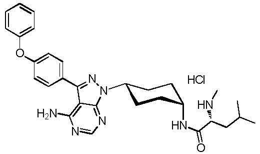

The synthesis of compound 109 was completed in the similar manner as described in example 76, except that trans-4-Boc-aminocyclohexanol was used in place of the raw material N-Boc-4-aminocyclohexanol. Exact Mass (calculated value): 527.66; MS(ESI) m/z(M+1)⁺: 528.3074.

### Example 110

### Synthesis of compound 110, (R)-2-amino-N-(4-(4-amino-3-(4-phenoxyphenyl) -1H-pyrazolo[3,4-d]pyrimidin-1-yl)cis-1,4-cyclohexyl)-4-methyl-2-(dimethylamino)pen tanamide

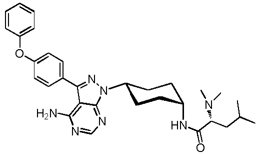

The synthesis of compound 110 was completed in the similar manner as described in example 76, except that trans-4-Boc-aminocyclohexanol was used in place of the raw material N-Boc-4-aminocyclohexanol. Exact Mass (calculated value): 541.69; MS(ESI) m/z(M+1)⁺: 542.3274.

### Example 111

### Synthesis of compound 111, (S)-2-amino-N-(4-(4-amino-3-(4-phenoxyphenyl)-1H -pyrazolo[3,4-d]pyrimidin-1-yl)cis-1,4-cyclohexyl)-4-methylpentanamide hydrochloride

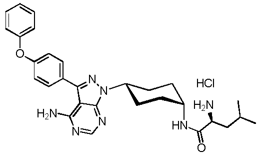

The synthesis of compound 111 was completed in the similar manner as described in example 76, except that trans-4-Boc-aminocyclohexanol was used in place of the raw material N-Boc-4-aminocyclohexanol. Exact Mass (calculated value): 513.63; MS(ESI) m/z(M+1)+: 514.2974.

### Example 112

### Synthesis of compound 112, (S)-2-amino-N-(4-(4-amino-3-(4-phenoxyphenyl)-1H-pyrazolo[3,4-d]pyrimidin-1-yl)cis-1,4-cyclohexyl)-4-methyl-2-(methylamino)pentanami de hydrochloride

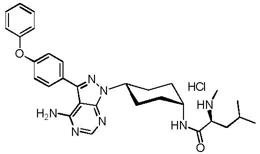

The synthesis of compound 112 was completed in the similar manner as described in example 76, except that trans-4-Boc-aminocyclohexanol was used in place of the raw material N-Boc-4-aminocyclohexanol. Exact Mass (calculated value): 527.66; MS(ESI) m/z(M+1)⁺: 528.3074.

### Example 113

### Synthesis of compound 113, (S)-2-amino-N-(4-(4-amino-3-(4-phenoxyphenyl)-1H-pyrazolo[3,4-d]pyrimidin-1-yl)cis-1,4-cyclohexyl)-4-methyl-2-(dimethylamino)pentana mide

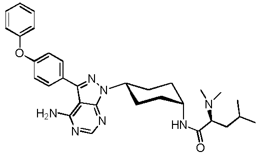

The synthesis of compound 113 was completed in the similar manner as described in example 76, except that trans-4-Boc-aminocyclohexanol was used in place of the raw material N-Boc-4-aminocyclohexanol. Exact Mass (calculated value): 541.69; MS(ESI) m/z(M+1)⁺: 542.3277.

### Example 114

### Synthesis of compound 114, (S)-2-amino-N-(4-(4-amino-3-(4-phenoxyphenyl)-1H-pyrazolo[3,4-d]pyrimidin-1-yl)trans-1,4-cyclohexyl)-4-methylpentanamide hydrochloride

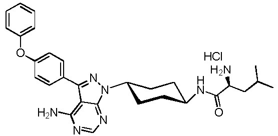

The synthesis of compound 114 was completed in the similar manner as described in example 76, except that cis-4-Boc-aminocyclohexanol was used in place of the raw material N-Boc-4-aminocyclohexanol. Exact Mass (calculated value): 513.63; MS(ESI) m/z(M+1)+: 514.2976.

### Example 115

### Synthesis of compound 115, (S)-2-amino-N-(4-(4-amino-3-(4-phenoxyphenyl)-1H-pyrazolo[3,4-d]pyrimidin-1-yl)trans-1,4-cyclohexyl)-4-methyl-2-(methylamino)pentana mide hydrochloride

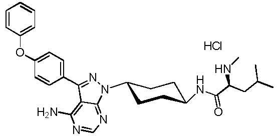

The synthesis of compound 115 was completed in the similar manner as described in example 76, except that cis-4-Boc-aminocyclohexanol was used in place of the raw material N-Boc-4-aminocyclohexanol. Exact Mass (calculated value): 527.66; MS(ESI) m/z(M+1)⁺: 528.3077.

### Example 116

### Synthesis of compound 116, (S)-2-amino-N-(4-(4-amino-3-(4-phenoxyphenyl)-1H-pyrazolo[3,4-d]pyrimidin-1-yl)trans-1,4-cyclohexyl)-4-methyl-2-(dimethylamino)penta namide

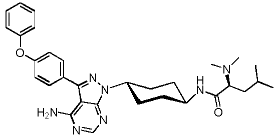

The synthesis of compound 116 was completed in the similar manner as described in example 76, except that cis-4-Boc-aminocyclohexanol was used in place of the raw material N-Boc-4-aminocyclohexanol. Exact Mass (calculated value): 541.69; MS(ESI) m/z(M+1)+: 542.3280.

### Example 117

### Synthesis of compound 117, (R)-2-amino-N-(4-(4-amino-3-(4-phenoxyphenyl)-1H -pyrazolo[3,4-d]pyrimidin-1-yl)trans-1,4-cyclohexyl)-4-methyl-2-(methylamino)pentan amide hydrochloride

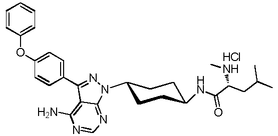

The synthesis of compound 117 was completed in the similar manner as described in example 76, except that cis-4-Boc-aminocyclohexanol was used in place of the raw material N-Boc-4-aminocyclohexanol. Exact Mass (calculated value): 527.66; MS(ESI) m/z(M+1)⁺: 528.3082.

### Example 118

### Synthesis of compound 118, (R)-2-amino-N-(4-(4-amino-3-(4-phenoxyphenyl)-1H -pyrazolo[3,4-d]pyrimidin-1-yl)trans-1,4-cyclohexyl)-4-methyl-2-(dimethylamino)penta namide

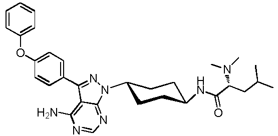

The synthesis of compound 118 was completed in the similar manner as described in example 76, except that cis-4-Boc-aminocyclohexanol was used in place of the raw material N-Boc-4-aminocyclohexanol. Exact Mass (calculated value): 541.69; MS(ESI) m/z(M+1)⁺: 542.3283.

### Example 119

### Synthesis of 119, (R)-2-amino-N-(4-(4-amino-3-(4-phenoxyphenyl)-1H-pyrazolo [3,4-d]pyrimidin-1-yl)cis-1,4-cyclohexyl)-2-(5-(tert-butyl)isoxazol-3-yl)acetamide hydrochloride

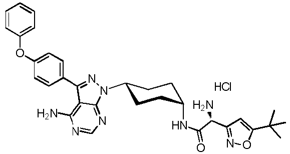

The synthesis of compound 119 was completed in the similar manner as described in example 76, except that trans-4-Boc-aminocyclohexanol was used in place of the raw material N-Boc-4-aminocyclohexanol. Exact Mass (calculated value): 580.68; MS(ESI) m/z(M+1)⁺: 581.2983.

### Example 120

### Synthesis of 120, (R)-2-amino-N-(4-(4-amino-3-(4-phenoxyphenyl)-1H-pyrazolo [3,4-d]pyrimidin-1-yl)cis-1,4-cyclohexyl)-2-(5-(tert-butyl)isoxazol-3-yl)-2-(methylamin o)acetamide hydrochloride

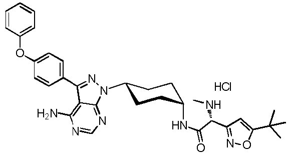

The synthesis of compound 120 was completed in the similar manner as described in example 76, except that trans-4-Boc-aminocyclohexanol was used in place of the raw material N-Boc-4-aminocyclohexanol. Exact Mass (calculated value): 594.71; MS(ESI) m/z(M+1)⁺: 595.3183.

### Example 121

### Synthesis of 121, (R)-2-amino-N-(4-(4-amino-3-(4-phenoxyphenyl)-1H-pyrazolo [3,4-d]pyrimidin-1-yl)cis-1,4-cyclohexyl)-2-(5-(tert-butyl)isoxazol-3-yl)-2-(dimethylam ino)acetamide

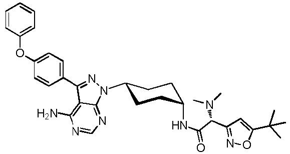

The synthesis of compound 121 was completed in the similar manner as described in example 76, except that trans-4-Boc-aminocyclohexanol was used in place of the raw material N-Boc-4-aminocyclohexanol. Exact Mass (calculated value): 608.73; MS(ESI) m/z(M+1)⁺: 609.3383.

### Example 122

### Synthesis of 122, N-(4-(4-amino-3-(4-phenoxyphenyl)-1H-pyrazolo[3,4-d] pyrimidin-1-yl)cis-1,4-cyclohexyl)-2-(5-(tert-butyl)isoxazol-3-yl)acetamide

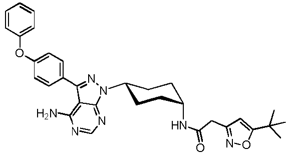

The synthesis of compound 122 was completed in the similar manner as described in example 76, except that trans-4-Boc-aminocyclohexanol was used in place of the raw material N-Boc-4-aminocyclohexanol. Exact Mass (calculated value): 565.67; (ESI) m/z(M+1)⁺: 566.2883.

### Example 123

### Synthesis of 123, N-(4-(4-amino-3-(4-phenoxyphenyl)-1H-pyrazolo[3,4-d] pyrimidin-1-yl)trans-1,4-cyclohexyl)-2-(5-(tert-butyl)isoxazol-3-yl)acetamide

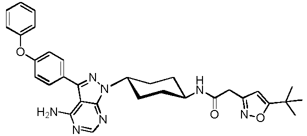

The synthesis of compound 123 was completed in the similar manner as described in example 76, except that cis-4-Boc-aminocyclohexanol was used in place of the raw material N-Boc-4-aminocyclohexanol. Exact Mass (calculated value): 565.67; MS(ESI) m/z(M+1)⁺: 566.2886.

### Example 124

### Synthesis of 124, (S)-2-amino-N-(4-(4-amino-3-(4-phenoxyphenyl)-1H-pyrazolo [3,4-d]pyrimidin-1-yl)trans-1,4-cyclohexyl)-2-(5-(tert-butyl)isoxazol-3-yl)acetamide hydrochloride

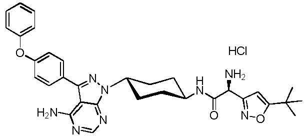

The synthesis of compound 124 was completed in the similar manner as described in example 76, except that cis-4-Boc-aminocyclohexanol was used in place of the raw material N-Boc-4-aminocyclohexanol. Exact Mass (calculated value): 580.68; MS(ESI) m/z(M+1)⁺: 581.2988.

### Example 125

### Synthesis of 125, (S)-2-amino-N-(4-(4-amino-3-(4-phenoxyphenyl)-1H-pyrazolo [3,4-d]pyrimidin-1-yl)trans-1,4-cyclohexyl)-2-(5-(tert-butyl)isoxazol-3-yl)-2-(methylam ino)acetamide hydrochloride

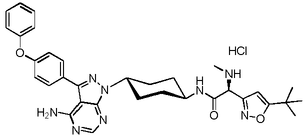

The synthesis of compound 125 was completed in the similar manner as described in example 76, except that cis-4-Boc-aminocyclohexanol was used in place of the raw material N-Boc-4-aminocyclohexanol. Exact Mass (calculated value): 594.71; MS(ESI) m/z(M+1)⁺: 595.3187.

### Example 126

### Synthesis of 126, (S)-2-amino-N-(4-(4-amino-3-(4-phenoxyphenyl)-1H-pyrazolo [3,4-d]pyrimidin-1-yl)trans-1,4-cyclohexyl)-2-(5-(tert-butyl)isoxazol-3-yl)-2-(dimethyla mino)acetamide

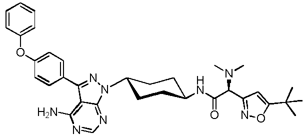

The synthesis of compound 126 was completed in the similar manner as described in example 76, except that cis-4-Boc-aminocyclohexanol was used in place of the raw material N-Boc-4-aminocyclohexanol. Exact Mass (calculated value): 608.73; MS(ESI) m/z(M+1)⁺: 609.3390.

### Example 127: Assay of inhibitory activity (enzyme activity) in vitro.

The IC₅₀ values of the compounds of the invention against the protein kinase RIPK2 (purchased from Promega) were determined in the assay of enzyme activity *in vitro.* 9 µL (6 ng/µL) of RIPK2 protein kinase was reacted with 1µL of testing compounds diluted in triplicate gradients for 4 h at room temperature (the final drug concentrations were 10 µM, 3 µM, 1 µM, 0.3 µM, 0.1 µM, 0.03 µM, 0.01 µM, 0.003 µM); 2 µL ATP and 3 µL substrate Poly (4:1 Glu, Tyr) Peptide (Promega, America) (the final concentrations were 10 µM and 0.2 µg/µL, respectively) were added and reacted for 1 h at 37 °C; after collecting 5 µL of kinase reaction solution, 5 µL ADP-Glo^{™} reagent (Promega, America) was added and allowed to react for 40 min at room temperature to terminate the kinase reaction and deplete the remaining ATP; 10 µL of kinase detection reagent was added to convert ADP to ATP; a coupled luciferase/luciferin reaction was used to detect newly synthesized ATP; after reading with Envision, graphs were plotted and IC₅₀ values were calculated (Table 2). The experiment results were shown in Table 2. The exemplary compounds of the invention have a potent inhibitory effect on RIPK2 protein kinase. These results indicate that the compounds of the invention are the inhibitors of RIPK2 kinase.

**Table 2 Testing results of the in vitro inhibitory activity (enzyme activity) against RIPK2 kinase**

| | RIPK2 (IC₅₀/nM) |
|---|---|
| Compound 3 | 0.09 |
| Compound 4 | 0.01 |
| Compound 21 | 0.076 |
| Compound 22 | 0.029 |
| Compound 23 | 0.015 |
| Compound 29 | 0.026 |
| Compound 30 | 0.022 |
| Compound 32 | 0.052 |
| Compound 33 | 0.027 |
| Compound 35 | 0.014 |
| Compound 39 | 0.047 |
| Compound 40 | 0.02 |
| Compound 41 | 0.03 |
| Compound 43 | 0.009 |
| Compound 44 | 0.007 |
| Compound 45 | 0.019 |
| Compound 46 | 0.011 |
| Compound 47 | 0.01 |
| Compound 48 | 0.006 |
| Compound 52 | 0.024 |
| Compound 72 | 0.006 |
| Compound 73 | 0.003 |
| Compound 76 | 0.007 |
| Compound 78 | 0.006 |

### Example 128: Therapeutic effect of compound 22 on rat colitis induced by enema administration

Sixty male Wister rats (purchased from Beijing Vital River Laboratory Animal Technology Co., Ltd.) were randomly divided into six groups, with 10 rats in each group: G1 was the blank control group; G2-G6 were the rat colitis model groups induced by rectal administration of DNBS solution (Tokyo Chemical Industry Co., Ltd., 50 mg/mL DNBS dissolved in 30% ethanol). Various animals were administered with drugs starting from two days before colitis induced by DNBS, and the administration schedules were shown in Table 3 below.

The induction of colitis was performed as follows. The rats were fasted for 40 h before administration, and were subcutaneously injected with 5% glucose saline (10 mg/kg, once a day) during the fast. On day 0, the fasted rats were anesthetized using Zoletil (intraperitoneal injection, 25 mg/kg, purchased from a pharmacy) and xylazine (intraperitoneal injection, 5 mg/kg, purchased from Sigma-Aldrich). In G2-G6 model groups, colitis was induced in rats by inserting a catheter from the anus into the left colic flexure (about 8 cm away from the anus) and administrating DNBS (0.5 mL per rat) through enema administration. The G1 blank control group was administered with 30% ethanol via enema using the same method. In order to avoid the reflux of enema fluid, animals with enema administration were kept in a head-down position for 15 mins, and then maintained in the Trendelenburg position until waking up.

**Table 3 Grouping and administration schedule**

| G r o u p | Test substance | Model | N u m b e r | R o u t e | Conce ntr-ation | Dosage | | Administration scheme |
|---|---|---|---|---|---|---|---|---|
| | | | | | mg/mL | mL/kg | mg/kg | |
| G1 | Blank-vehicle control vehicle: citrate solution | Ethanol | 10 | p.o. | N/A | 10 | N/A | QD -2 to 6 days |
| G2 | Model-vehicle control vehicle: citrate solution | DNBS | 10 | p.o. | N/A | 10 | N/A | QD -2 to 6 days |
| G3 | Model-GSK2983559 (purchased from MCE, Shanghai) | DNBS | 10 | p.o. | 1 | 10 | 10 | BID -2 to 6 days Administration on the day of molding, 2 h before molding, and 10 h after molding |
| G4 | Model-compound 22 | DNBS | 10 | p.o. | 1 | 10 | 10 | BID -2 to 6 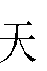 Administration on the day of molding, 2 h before molding, and 10 h after molding |
| G5 | Model-compound 22 | DNBS | 10 | p.o. | 0.5 | 10 | 5 | QD -2 to 6 days Administration on the day of molding, 2 h before molding |
| G6 | Model-compound 22 | DNBS | 10 | p.o. | 2 | 10 | 20 | QD -2 to 6 days Administration on the day of molding, 2 h before molding |

During the administration period, the body weights of rats in all groups were recorded every day. Six days later, the animals were euthanized and the colorectal tissues were collected. The ulcer area of the colorectum, as well as the degrees of adhesion, intestinal obstruction, ulceration, and intestinal wall thickening, were measured. The pathological condition of the intestinal wall was evaluated. The conditions of colon damage was evaluated according to Table 4.

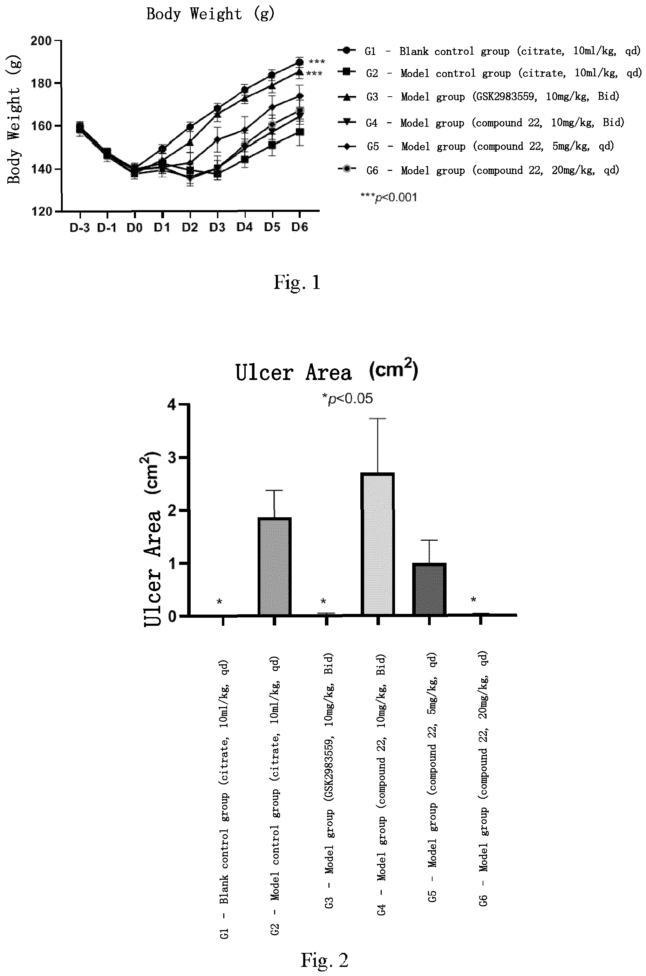

If the ulcer is irregular in shape, it can be considered as a rectangle, and then the length and width were measured to evaluate the ulcer area. Ulcer area (cm²) = ulcer length (cm) × ulcer width (cm). The results of ulcer areas for each group are shown in Figure 2.

**Table 4 Macroscopic evaluation of colonic ulcer scores of rats**

| Project | Score |
|---|---|
| Degree of adhesion: | |
| No adhesion | 0 |
| Mild adhesion | 1 |
| Severe adhesion | 2 |

| Degree of intestinal obstruction: | |
|---|---|
| None | 0 |
| Mild obstruction | 1 |
| Severe obstruction | 2 |

| Degree of ulceration: | |
|---|---|
| None | 0 |
| One ulcer with a length <1 cm | 1 |
| Two ulcers with lengths <1 cm | 2 |
| Three or more ulcer areas, or one of them >1 cm | 3 |
| Degree of intestinal wall thickening: | |
| < 1mm | 0 |
| 1-3mm | 1 |
| Greater than 3mm | 2 |

As shown in Figures 1-3, the results for the positive drug subjects (GSK2983559, 10 mg/kg) (G3) which were administered BID to the inflammatory colitis model animals revealed that, the body weights, the macroscopical ulcer areas of colorectal tissues, as well as the scores for the degrees of adhesion, intestinal obstruction, ulceration, and intestinal wall thickening of the experimental animals of G3 group, were significantly lower than those for the G2 group (P<0.05). When the compound 22 of the invention was administered at a dose of 20 mg/kg (G6 group), the animal body weights, the ulcer areas of colorectal tissues, as well as the scores for the degrees of adhesion, intestinal obstruction, ulceration, intestinal wall thickening, were also significantly lower than those for the G2 group, and comparable to those for the positive control group. It demonstrated that the compounds of the invention have an positive therapeutic effect on the inflammatory bowel disease model.

### Example 129: Therapeutic effect of compound 22 on the rat arthritis model

Experimental animals: Fifty male Sprague-Dawley (SD) rats weighing 180±20 g (provided by Qinglongshan Animal Breeding Center, license number SCXK(Su)2017-0001) were fed with conventional pellet food (Jiangsu Xietong Biology Co., Ltd.) and housed in a clean animal facility. The rats were housed under a 12 h dark/12 h light cycle with free access to food and water. The temperature was maintained between 20 °C to 26 °C, while the relative humidity ranged from 40% to 70%. Fifty SD rats were divided into five groups, including control group, vehicle group, positive control group (Meloxicam) (purchased from pharmacy), compound 22 at 10 mg/kg group, compound 22 at 30 mg/kg group. Each group of 10 animals was administered QD for a continuous period of 21 days.

The models were constructed according to the methods described in the *Production and Application of Animal Models for Pharmaceutic Experiments,* and the *Standard Operating Procedured for Constructiion of Adjuvant Arthritis Model* at the Model Animal Center. The method was decribed below.

### Adjuvant Emulsification

1) Bacillus Calmette-Guerin (BCG) vaccine (purchased from Difco, USA, H37Ra) was inactivated in 80°C water bath for 2 h, and then was dissolved in Freund's adjuvant (purchased from Sigma-Aldrich, USA) to prepare 10 mg/ml solution; 2) a 5 ml sterile syringe was used to taken up 3 ml Freund's complete adjuvant BCG vaccine solution, and another 5 ml sterile syringe was used to taken up 3 ml Freund's complete adjuvant BCG vaccine solution; 3) a disposable sterile three-way valve was connected, and the syringes were repeatedly pushed to mix the adjuvant; 4) after being sufficiently emulsified, the emulsified adjuvant was dropped into water, and the droplets that remained stable for at least 30 seconds without diffusion were considered as fully emulsified.

### Model Construction

1) 10% Chloral hydrate (purchased from Sigma-Aldrich, USA) was injected intraperitoneally into rats at a dose of 0.3ml/100g for anesthesia; 2) after the rats were anesthetized, the emulsified adjuvant was taken up using a 1 ml sterile syringe, and then was injected intradermally into the right hind paw of each rat at a dose of 0.1 ml; 3) After the injection, the paw was applied pressure for 30 seconds to prevent adjuvant spillage, and then 75% ethanol was used for sterilization; 4) the paws of rats in the control group were subcutaneously injected with the same volume of normal saline as a blank control, which was considered as the first day of modeling; 5) the modeled rats were housed in rearing cages and fed conventionally.

After 7 days of modeling, each rat was intragastrically given drugs QD. The control group and the vehicle group were intragastrically given an equal volume of citrate solution everyday.

Paw swelling measurement: 1) the volume of right hind paw for the rats in each group was measured and recorded using a plethysmometer for paw volume before administration, which was used as the baseline value before modeling; 2) the volume of the rats' paw was measured at the end of administration (Day 21), which was used as the secondary value; 3) the swelling volume of the rats' paw was calculated according to the following formula: Δml = paw volume after induction of inflammation (ml) - paw volume before induction of inflammation (ml)

The arthritis index scores were determined as following: at the end of administration (Day 21), rats in each group were evaluated according to the scoring standard described in Table 5. The arthritis index score was the cumulative score of lesion severity in the right hind paw of each rat. The maximum score for four paws was 16.

**Table 5 Standards for arthritis index scores of the paws**

| Score | Symptoms |
|---|---|
| 0 | No swelling |
| 1 | Slight red and swollen at ankle joint |
| 2 | Slight red and swollen from ankle joint to toe joint or at metacarpal joint |
| 3 | Moderate red and swollen from ankle joint to toe joint or at metacarpal joint |
| 4 | Severe red and swollen from ankle joint to toe joint or at metacarpal joint |

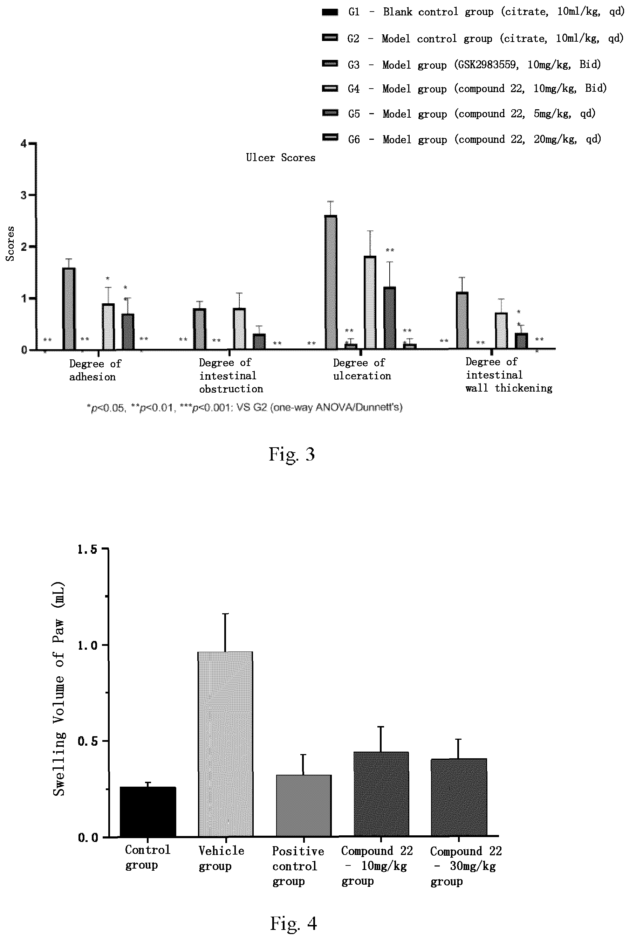

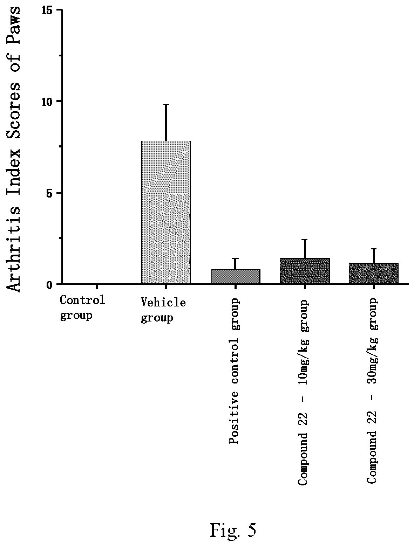

The results were shown in Figure 4 and Figure5. When administered orally at doses of 10 mg/kg and 30 mg/kg, compound 22 significantly alleviated paw swelling in the adjuvant-induced arthritis model rats.

### Industrial Application

The invention provides a RIPK2 kinase inhibitor compound, which can be used to reduce or inhibit the activity of RIPK2 kinase in cells or subjects, and/or prevent or treat RIPK2-related diseases in subjects, such as hypersensitivity reactions or autoimmune inflammatory-related diseases. Therefore, it can be made into corresponding drugs, which is applicable in for industrial application.

Although the invention is described in detail herein, the invention is not limited to the same. One skilled in the art can make modification according to the principle of the invention. Therefore, all modifications made based on the principle of the invention should be understood to be within the protection scope of the invention.

## Claims

1. Use of a compound of formula (I) or a pharmaceutically acceptable salt, solvate, isomer, ester, acid, metabolite, or prodrug thereof for treating or preventing hypersensitivity reactions or autoimmune inflammatory-related diseases: 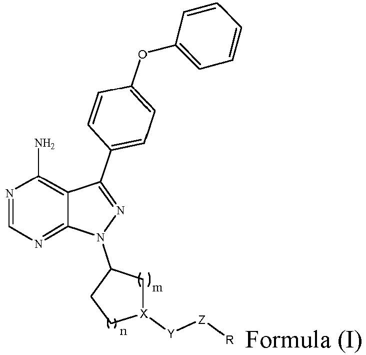 wherein:
m is selected from the integer of 1 or 2;
n is selected from the integer of 0-4;
X is N or CH, and when X is N, Y is a chemical bond; when X is CH, Y is NH;
Z is selected from CH₂, 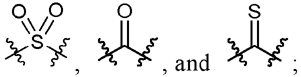
R is selected from the group consisting of amino, unsubstituted or substituted C1-C8 alkyl with carbon atom(s) being optionally substituted by 1-3 independent R1, unsubstituted or substituted C3-C8 cycloalkyl with carbon atom(s) being optionally substituted by 1-3 independent R1, unsubstituted or substituted C1-C8 haloalkyl with carbon atom(s) being optionally substituted by 1-3 independent R1, unsubstituted or substituted C1-C8 aminoalkyl with carbon atom(s) being optionally substituted by 1-3 independent R1 or heteroatom(s) being optionally substituted by R2, unsubstituted or substituted C1-C8 cyanoalkyl with carbon atom(s) being optionally substituted by 1-3 independent R1, unsubstituted or substituted C1-C8 hydroxyalkyl with carbon atom(s) being optionally substituted by 1-3 independent R1, unsubstituted or substituted C1-C8 alkoxy with carbon atom(s) being optionally substituted by 1-3 independent R1, unsubstituted or substituted C3-C8 heterocyclyl with carbon atom(s) being optionally substituted by 1-3 independent R1 or heteroatom(s) being optionally substituted by R2, unsubstituted or substituted aryl with carbon atom(s) being optionally substituted by 1-3 independent R1, unsubstituted or substituted heteroaryl with carbon atom(s) being optionally substituted by 1-3 independent R1 or heteroatom(s) being optionally substituted by R2, carbamoyl, unsubstituted or substituted C1-C8 alkylformyl with carbon atom(s) being optionally substituted by 1-3 independent R1, unsubstituted or substituted C3-C8 cycloalkylformyl with carbon atom(s) being optionally substituted by 1-3 independent R1, unsubstituted or substituted C3-C8 heterocyclyl formyl with carbon atom(s) being optionally substituted by 1-3 independent R1 or heteroatom(s) being optionally substituted by R2, unsubstituted or substituted arylformyl with carbon atom(s) being optionally substituted by 1-3 independent R1, unsubstituted or substituted C1-C8 alkylamino(C1-C8)alkyl with carbon atom(s) being optionally substituted by 1-3 independent R1, unsubstituted or substituted C1-C8 alkyl(C3-C6 cycloalkyl) with carbon atom(s) being optionally substituted by 1-3 independent R1, unsubstituted or substituted C3-C6 cycloalkyl(C1-C8 alkyl) with carbon atom(s) being optionally substituted by 1-3 independent R1, unsubstituted or substituted C1-C8 alkyl(C3-C6 heterocyclyl) with carbon atom(s) being optionally substituted by 1-3 independent R1 or heteroatom(s) being optionally substituted by R2, unsubstituted or substituted C3-C6 heterocyclyl(C1-C8 alkyl) with carbon atom(s) being optionally substituted by 1-3 independent R1 or heteroatom(s) being optionally substituted by R2, unsubstituted or substituted C1-C8 alkyl(aryl) with carbon atom(s) being optionally substituted by 1-3 independent R1, unsubstituted or substituted C1-C8 alkyl(heteroaryl) with carbon atom(s) being optionally substituted by 1-3 independent R1 or heteroatom(s) being optionally substituted by R2, and unsubstituted or substituted C1-C8 aminoalkyl(carbamoyl) with carbon atom(s) being optionally substituted by 1-3 independent R1;
R1 is selected from the group consisting of halogen, amino, nitro, cyano, hydroxyl, C1-C8 alkyl, C3-C8 cycloalkyl, C1-C8 alkoxy, aryl, heteroaryl with heteroatom(s) being optionally substituted by R2, C1-C8 alkoxy carbonyl, C1-C8 alkyl(heteroaryl) with heteroatom(s) being optionally substituted by R2, and C1-C8 alkyl(C3-C6 heterocyclyl) with heteroatom(s) being optionally substituted by R2;
R2 is selected from the group consisting of amino protecting groups, C1-C8 alkyl, and C1-C8 alkoxy carbonyl, wherein the amino protecting groups are independently selected from the group consisting of tert-butoxycarbonyl, benzyloxycarbonyl, 9-fluorenylmethoxycarbonyl, benzyl, and para-methoxyphenyl.

2. Use of the compound or a pharmaceutically acceptable salt, solvate, isomer, ester, acid, metabolite, or prodrug thereof of claim 1, wherein n is 1 or 2.

3. Use of the compound or a pharmaceutically acceptable salt, solvate, isomer, ester, acid, metabolite, or prodrug thereof of claim 1, wherein
Z is

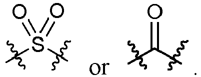

4. Use of the compound or a pharmaceutically acceptable salt, solvate, isomer, ester, acid, metabolite, or prodrug thereof of claim 1, wherein
R is selected from the group consisting of substituted or unsubstituted C1-C6 alkyl, C1-C4 haloalkyl, C3-C6 cycloalkyl, amino, carbamoyl, C1-C6 aminoalkyl, heteroaryl, C3-C6 heterocyclyl, aryl, C1-C4 cyanoalkyl, di(C1-C4 alkyl)-N-(C1-C4)alkyl, C1-C6 hydroxyalkyl, C1-C4 alkyl(C3-C6 heterocyclyl), C1-C4 alkyl(C3-C6 cycloalkyl), C1-C4 aminoalkyl(carbamoyl), C1-C4 alkyl(heteroaryl), and C1-C4 alkyl(aryl).

5. Use of the compound or a pharmaceutically acceptable salt, solvate, isomer, ester, acid, metabolite, or prodrug thereof of claim 1, wherein
m is 1, X is N, Y is a chemical bond, and the compound is represented by formula (II):

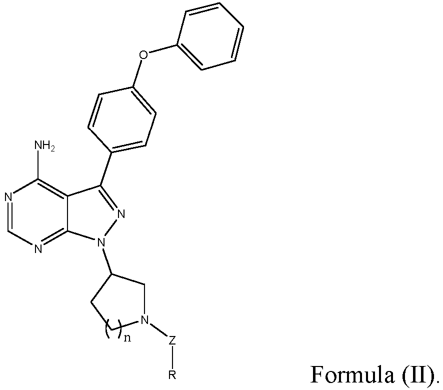

6. Use of the compound or a pharmaceutically acceptable salt, solvate, isomer, ester, acid, metabolite, or prodrug thereof of claim 1, wherein
m is 2, X is N, Y is a chemical bond, and the compound is represented by formula (III):

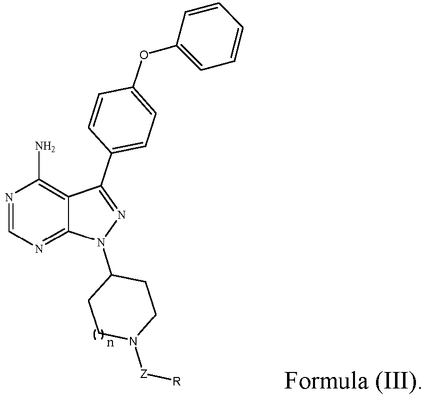

7. Use of the compound or a pharmaceutically acceptable salt, solvate, isomer, ester, acid, metabolite, or prodrug thereof of claim 1, wherein
m is 2, X is CH, Y is NH, and the compound is represented by formula (IV):

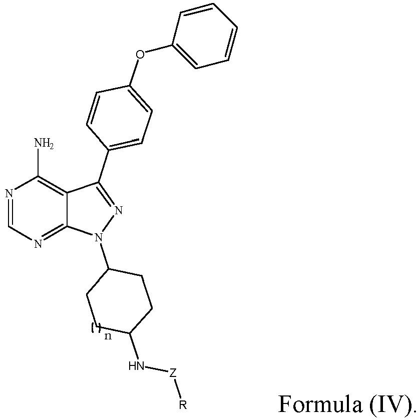

8. Use of the compound or a pharmaceutically acceptable salt, solvate, isomer, ester, acid, metabolite, or prodrug thereof of claim 1, wherein the compound is selected from the group consisting of:
(R)-(3-(4-amino-(4-phenoxyphenyl)-1H-pyrazolo[3,4-d]pyrimidin-1-yl)piperidin-1 -yl)(cyclopropyl) methanone (1);
(R)-1-(3-(4-amino-(4-phenoxyphenyl)-1H-pyrazolo[3,4-d]pyrimidin-1-yl)piperidin -1-yl)-2,2,2-trifluoroethanone (2);
(R)-3-(4-phenoxyphenyl)-1-(1-((trifluoromethyl)sulfonyl)piperidin-3-yl)-1H-pyraz olo[3,4-d]pyrimidin-4-amine (3);
(R)-3-(4-amino-(4-phenoxyphenyl)-1H-pyrazolo[3,4-d]pyrimidin-1-yl)piperidin-1-sulfonamide (4);
(R)-2-(3-(4-amino-(4-phenoxyphenyl)-1H-pyrazolo[3,4-d]pyrimidin-1-yl)piperidin -1-yl)-2-oxamide (5);
(S)-2-amino-1-((R)-3-(4-amino-(4-phenoxyphenyl)-1H-pyrazolo[3,4-d]pyrimidin-1-yl)piperidin-1-yl)-3-(1H-imidazol-4-yl)propan-1-one (6);
(R)-2-amino-1-((R)-3-(4-amino-(4-phenoxyphenyl)-1H-pyrazolo[3,4-d]pyrimidin-1-yl)piperidin-1-yl)-3-(1H-imidazol-4-yl) propan-1-one (7);
(R)-2-amino-1-((R)-3-(4-amino-(4-phenoxyphenyl)-1H-pyrazolo[3,4-d]pyrimidin-1-yl)piperidin-1-yl)-3-(1H-indol-3-yl) propan-1-one (8);
(S)-2-amino-1-((R)-3-(4-amino-(4-phenoxyphenyl)-1H-pyrazolo[3,4-d]pyrimidin-1-yl)piperidin-1-yl)-3-(1H-indol-3-yl)propan-1-one (9);
(R)-2-amino-1-((R)-3-(4-amino-(4-phenoxyphenyl)-1H-pyrazolo[3,4-d]pyrimidin-1-yl)piperidin-1-yl)-3-(4-hydroxyphenyl)propan-1-one (10);
(S)-2-amino-1-((R)-3-(4-amino-(4-phenoxyphenyl)-1H-pyrazolo[3,4-d]pyrimidin-1-yl)piperidin-1-yl)-3-(4-hydroxyphenyl)propan-1-one (11);
(S)-2-amino-1-((R)-3-(4-amino-(4-phenoxyphenyl)-1H-pyrazolo[3,4-d]pyrimidin-1-yl)piperidin-1-yl)-4-methylpentan-1-one (12);
(R)-(3-(4-amino-(4-phenoxyphenyl)-1H-pyrazolo[3,4-d]pyrimidin-1-yl)piperidin-1 -yl)(pyridin-3-yl)methanone (13);
(R)-(3-(4-amino-(4-phenoxyphenyl)-1H-pyrazolo[3,4-d]pyrimidin-1-yl)piperidin-1 -yl)(piperazin-1-yl)methanone (14);
(R)-(3-(4-amino-(4-phenoxyphenyl)-1H-pyrazolo[3,4-d]pyrimidin-1-yl)piperidin-1 -yl)(pyrimidin-5-yl)methanone (15);
methyl (R)-4-(3-(4-amino-(4-phenoxyphenyl)-1H-pyrazolo[3,4-d]pyrimidin-1-yl) piperidin-1-carbonyl)benzoate (16)
(R)-(3-(4-amino-(4-phenoxyphenyl)-1H-pyrazolo[3,4-d]pyrimidin-1-yl)piperidin-1 -yl)(6-aminopyridin-3-yl)methanone (17);
(R)-(3-(4-amino-(4-phenoxyphenyl)-1H-pyrazolo[3,4-d]pyrimidin-1-yl)piperidin-1 -yl)-benzo[1,3]dioxol-5-yl-methanone (18);
(R)-(3-(4-amino-(4-phenoxyphenyl)-1H-pyrazolo[3,4-d]pyrimidin-1-yl)piperidin-1 -yl)-piperidin-4-yl-methanone (19);
(R)-3-(3-(4-amino-(4-phenoxyphenyl)-1H-pyrazolo[3,4-d]pyrimidin-1-yl)piperidin -1-yl)-3-oxo-propionitrile (20);
(R)-(3-(4-amino-(4-phenoxyphenyl)-1H-pyrazolo[3,4-d]pyrimidin-1-yl)piperidin-1 -yl)(2-aminopyrimidin-5-yl)methanone (21);
(R)-1-(3-(4-amino-(4-phenoxyphenyl)-1H-pyrazolo[3,4-d]pyrimidin-1-yl)piperidin -1-yl)-2-(dimethylamino)ethanone (22);
1-((R)-3-(4-amino-(4-phenoxyphenyl)-1H-pyrazolo[3,4-d]pyrimidin-1-yl)piperidin -1-yl)-2-hydroxypropan-1-one (23);
(R)-2-amino-1-((R)-3-(4-amino-(4-phenoxyphenyl)-1H-pyrazolo[3,4-d]pyrimidin-1-yl)piperidin-1-yl)propan-1-one (24);
(2R,3R)-2-amino-1-((R)-3-(4-amino-(4-phenoxyphenyl)-1H-pyrazolo[3,4-d]pyrimi din-1-yl) piperidin-1-yl)-3- methylpentan-1-one (25);
(R)-2-amino-1-((R)-3-(4-amino-(4-phenoxyphenyl)-1H-pyrazolo[3,4-d]pyrimidin-1-yl) piperidin-1-yl)-3- phenylpropan-1-one (26);
(R)-2-amino-1-((R)-3-(4-amino-(4-phenoxyphenyl)-1H-pyrazolo[3,4-d]pyrimidin-1-yl) piperidin-1-yl)-4-methylpentan-1-one (27);
(R)-2-amino-1-((R)-3-(4-amino-(4-phenoxyphenyl)-1H-pyrazolo[3,4-d]pyrimidin-1-yl)pyrrolidin-1-yl)-4- methylpentan-1-one (28);
(R)-(3-(4-amino-(4-phenoxyphenyl)-1H-pyrazolo[3,4-d]pyrimidin-1-yl)piperidin-1 -yl)(isoxazol-5-yl) methanone (29);
(R)-1-(1-methylsulfonyl)piperidin-3-yl)-3-(4-phenoxyphenyl)-1H-pyrazolo[3,4-d]p yrimidin-4-amine (30);
(R)-3-(4-amino-(4-phenoxyphenyl)-1H-pyrazolo[3,4-d]pyrimidin-1-yl)piperidin-1-yl)-2-morpholinyl ethanone (31);
(R)-3-(4-amino-(4-phenoxyphenyl)-1H-pyrazolo[3,4-d]pyrimidin-1-yl)piperidin-1-yl)(cyclopentan-2-yl) ethanone (32);
(R)-(3-(4-amino-(4-phenoxyphenyl)-1H-pyrazolo[3,4-d]pyrimidin-1-yl)piperidin-1 -yl)(1-methylpiperidin-4-yl)methanone (33);
(R)-3-(4-amino-(4-phenoxyphenyl)-1H-pyrazolo[3,4-d]pyrimidin-1-yl)piperidin-1-yl)ethanone (34);
(R)-(3-(4-amino-(4-phenoxyphenyl)-1H-pyrazolo[3,4-d]pyrimidin-1-yl)piperidin-1 -yl)(4-((4-methylpiperazin-1-yl)methyl)phenyl)methoanone (35)
(R)-3-(4-amino-(4-phenoxyphenyl)-1H-pyrazolo[3,4-d]pyrimidin-1-yl)piperidin-1-yl)-3-chloroacetone (36);
(R)-3-(4-amino-(4-phenoxyphenyl)-1H-pyrazolo[3,4-d]pyrimidin-1-yl)piperidin-1-yl)-2-bromoacetone (37);
(R)-3-(4-amino-(4-phenoxyphenyl)-1H-pyrazolo[3,4-d]pyrimidin-1-yl)piperidin-1-yl)-2-chloroacetone (38);
(R)-3-(4-amino-(4-phenoxyphenyl)-1H-pyrazolo[3,4-d]pyrimidin-1-yl)piperidin-1-yl)-2-(4-methylpiperazin-1-yl)ethanone (39);
(R)-3-(4-phenoxyphenyl)-1-(1-(propylsulfonyl)piperidin-3-yl)-1H-pyrazolo[3,4-d] pyrimidin-4-amine (40);
(R)-3-(4-phenoxyphenyl)-1-(1-(ethylsulfonyl)piperidin-3-yl)-1H-pyrazolo[3,4-d]p yrimidin-4-amine (41);
(R)-3-(4-amino-(4-phenoxyphenyl)-1H-pyrazolo[3,4-d]pyrimidin-1-yl)piperidin-1-yl)trichloroethanone (42)
(R)-2-amino-1-(3-(4-amino-(4-phenoxyphenyl)-1H-pyrazolo[3,4-d]pyrimidin-1-yl) piperidin-1-yl)ethanone (43);
(R)-2-amino-1-((R)-3-(4-amino-(4-phenoxyphenyl)-1H-pyrazolo[3,4-d]pyrimidin-1-yl)piperidin-1-yl)-3-hydroxybutanone (44);
(R)-2-amino-1-((R)3-(4-amino-(4-phenoxyphenyl)-1H-pyrazolo[3,4-d]pyrimidin-1 -yl)piperidin-1-yl)-3-methlybutanone (45);
(R)-3-amino-1-(3-(4-amino-(4-phenoxyphenyl)-1H-pyrazolo[3,4-d]pyrimidin-1-yl) piperidin-1-yl)acetone (46);
(R)-2-amino-1-((R)-(3-(4-amino-(4-phenoxyphenyl)-1H-pyrazolo[3,4-d]pyrimidin-1-yl)piperidin-1-yl)acetone (47);
(R)-2-amino-1-((R)-(3-(4-amino-(4-phenoxyphenyl)-1H-pyrazolo[3,4-d]pyrimidin-1-yl)piperidin-1-yl)-3-hydroxyacetone (48);
(R)-3-(4-amino-(4-phenoxyphenyl)-1H-pyrazolo[3,4-d]pyrimidin-1-yl)piperidin-1-yl)-3,3-dimethyl butanone (49);
(R)-3-(4-amino-(4-phenoxyphenyl)-1H-pyrazolo[3,4-d]pyrimidin-1-yl)piperidin-1-yl)-2,2-dimethyl butanone (50);
(R)-3-(4-amino-(4-phenoxyphenyl)-1H-pyrazolo[3,4-d]pyrimidin-1-yl)piperidin-1-yl)-2,2,3-trifluoroacetone (51);
(R)-3-(4-amino-(4-phenoxyphenyl)-1H-pyrazolo[3,4-d]pyrimidin-1-yl)piperidin-1-yl)-2-cyclopropyl ethanone (52);
(R)-3-(4-amino-(4-phenoxyphenyl)-1H-pyrazolo[3,4-d]pyrimidin-1-yl)piperidin-1-yl)-3-methyl butanone (53);
(R)-3-(4-amino-(4-phenoxyphenyl)-1H-pyrazolo[3,4-d]pyrimidin-1-yl)piperidin-1-yl)butanone (54);
(R)-3-(4-amino-(4-phenoxyphenyl)-1H-pyrazolo[3,4-d]pyrimidin-1-yl)piperidin-1-yl)-2,2-dimethylacetone (55);
(S)-4-amino-5-((R)-3-(4-amino-(4-phenoxyphenyl)-1H-pyrazolo[3,4-d]pyrimidin-1-yl)piperidin-1-yl)-5-oxo-pentanamide (56);
(R)-4-amino-5-((R)-3-(4-amino-(4-phenoxyphenyl)-1H-pyrazolo[3,4-d]pyrimidin-1-yl)piperidin-1-yl)-5-oxo-pentanamide (57);
(R)-1-(3-(4-amino-(4-phenoxyphenyl)-1H-pyrazolo[3,4-d]pyrimidin-1-yl)piperidin -1-yl)-3-(4-methylpiperazin-1-yl)acetone (58);
tert-butyl (R)-(2-(3-(4-amino-3-(4-phenoxyphenyl)-1H-pyrazolo[3,4-d]pyrimidin -1-yl)piperidin-1-yl)-2-carbonyl)ethyl carbamate (59);
methyl (R)-4-(3-(4-amino-(4-phenoxyphenyl)-1H-pyrazolo[3,4-d]pyrimidin-1-yl) piperidin-1-carbonyl)piperidin-1-formate (60);
(R)-1-((R)-3-(4-amino-(4-phenoxyphenyl)-1H-pyrazolo[3,4-d]pyrimidin-1-yl)piper idin-1-yl)-2-hydroxyl-4-methylpentan-1-one (61);
(R)-3-(4-amino-(4-phenoxyphenyl)-1H-pyrazolo[3,4-d]pyrimidin-1-yl)piperidin-1-yl)-5-aminopentanone (62);
(R)-3-(4-amino-(4-phenoxyphenyl)-1H-pyrazolo[3,4-d]pyrimidin-1-yl)piperidin-1-yl)-2-hydroxyhexan-1-one (63);
(R)-3-(4-amino-(4-phenoxyphenyl)-1H-pyrazolo[3,4-d]pyrimidin-1-yl)pyrrolidin-1 -yl)-2-aminoethanone (64);
(R)-3-(4-amino-(4-phenoxyphenyl)-1H-pyrazolo[3,4-d]pyrimidin-1-yl)pyrrolidin-1 -yl)-2-(dimethylamino)ethanone (65);
tert-butyl (R)-(2-(3-(4-amino-(4-phenoxyphenyl)-1H-pyrazolo[3,4-d]pyrimidin-1-yl)pyrrolidin-1-yl)-2-carbonyl) ethylcarbamate (66);
(R)-1-(1-(methylsulfonyl)pyrrolidin-3-yl)-3-(4-phenoxyphenyl)-1H-pyrazolo[3,4-d ]pyrimidin-4-amine (67);
(4-(4-amino-(4-phenoxyphenyl)-1H-pyrazolo[3,4-d]pyrimidin-1-yl)piperidin-1-yl)( 1-methylpiperidin-4-yl) methanone (68);
1-(4-(4-amino-(4-phenoxyphenyl)-1H-pyrazolo[3,4-d]pyrimidin-1-yl)piperidin-1-y l)-2-(4-methylpiperazin-1-yl)ethanone (69);
1-(4-(4-amino-(4-phenoxyphenyl)-1H-pyrazolo[3,4-d]pyrimidin-1-yl)piperidin-1-y l)-2-morpholinyl-ethanone (70);
(4-(4-amino-(4-phenoxyphenyl)-1H-pyrazolo[3,4-d]pyrimidin-1-yl)piperidin-1-yl)-(4-((4-methylpiperazin-1-yl)methyl)phenyl)methanone (71);
1-(4-(4-amino-(4-phenoxyphenyl)-1H-pyrazolo[3,4-d]pyrimidin-1-yl)piperidin-1-y l)-2-(dimethylamino)ethanone (72);
1-(1-(methylsulfonyl)piperidin-4-yl)-3-(4-phenoxyphenyl)-1H-pyrazolo[3,4-d]pyri midin-4-amine (73);
(R)-2-amino-1-(4-(4-amino-(4-phenoxyphenyl)-1H-pyrazolo[3,4-d]pyrimidin-1-yl) piperidin-1-yl)-4-methylpentan-1-one (74);
1-(4-(4-amino-(4-phenoxyphenyl)-1H-pyrazolo[3,4-d]pyrimidin-1-yl)piperidin-1-y l)-2-(methylamino)ethanone (75);
N-(4-(4-amino-(4-phenoxyphenyl)-1H-pyrazolo[3,4-d]pyrimidin-1-yl)cyclohexyl)-2-(dimethylamino)acetamide (76);
(R)-2-amino-N-(4-(4-amino-3-(4-phenoxyphenyl)-1H-pyrazolo[3,4-d]pyrimidin-1-yl)cyclohexyl)-4-methyl-pentanamide hydrochloride (77);
N-(4-(4-amino-3-(4-phenoxyphenyl)-1H-pyrazolo[3,4-d]pyrimidin-1-yl)cyclohexy l)methylsulfonamide (78);
N-(4-(4-amino-3-(4-phenoxyphenyl)-1H-pyrazolo[3,4-d]pyrimidin-1-yl)cyclohexy l)-2-(methylamino)acetamide (79);
N-(4-(4-amino-3-(4-phenoxyphenyl)-1H-pyrazolo[3,4-d]pyrimidin-1-yl)cyclohexy l)-1-methylaminopiperidin-4-formamide (80);
N-(4-(4-amino-3-(4-phenoxyphenyl)-1H-pyrazolo[3,4-d]pyrimidin-1-yl)cyclohexy l)-2-(4-methylpiperazin-1-yl)acetamide (81);
N-(4-(4-amino-3-(4-phenoxyphenyl)-1H-pyrazolo[3,4-d]pyrimidin-1-yl)cyclohexy l)-2-morpholinyl-acetamide (82);
N-(4-(4-amino-3-(4-phenoxyphenyl)-1H-pyrazolo[3,4-d]pyrimidin-1-yl)cyclohexy l)-2-cyanoacetamide (83);
(R)-2-amino-N-(4-(4-amino-3-(4-phenoxyphenyl)-1H-pyrazolo[3,4-d]pyrimidin-1-yl)cis-1,4-cyclohexyl)-4-methyl-pentanamide hydrochloride (84);
(R)-2-amino-N-(4-(4-amino-3-(4-phenoxyphenyl)-1H-pyrazolo[3,4-d]pyrimidin-1-yl)trans-1,4-cyclohexyl)-4-methyl pentanamide hydrochloride (85);
(R)-2-amino-N-(4-(4-amino-(4-phenoxyphenyl)-1H-pyrazolo[3,4-d]pyrimidin-1-yl )cyclohexyl)-3-methyl butyramide hydrochloride (86);
(R)-N-(4-(4-amino-(4-phenoxyphenyl)-1H-pyrazolo[3,4-d]pyrimidin-1-yl)cyclohe xyl)-3-methyl-2-(methylamino)butyramide hydrochloride (87);
(R)-N-(4-(4-amino-(4-phenoxyphenyl)-1H-pyrazolo[3,4-d]pyrimidin-1-yl)cyclohe xyl)-2-(dimethylamino)-3-methylbutyramide (88);
(S)-2-amino-N-(4-(4-amino-(4-phenoxyphenyl)-1H-pyrazolo[3,4-d]pyrimidin-1-yl )cyclohexyl)-3-methylbutyramide hydrochloride (89);
(S)-N-(4-(4-amino-(4-phenoxyphenyl)-1H-pyrazolo[3,4-d]pyrimidin-1-yl)cyclohex yl)-3-methyl-2-(methylamino)butyramide hydrochloride (90);
(S)-N-(4-(4-amino-(4-phenoxyphenyl)-1H-pyrazolo[3,4-d]pyrimidin-1-yl)cyclohex yl)- 2-(dimethylamino)-3-methylbutyramide (91);
(R)-N-(4-(4-amino-(4-phenoxyphenyl)-1H-pyrazolo[3,4-d]pyrimidin-1-yl)cyclohe xyl)- 4-methyl-2-(methylamino)pentanamide hydrochloride (92);
(R)-N-(4-(4-amino-(4-phenoxyphenyl)-1H-pyrazolo[3,4-d]pyrimidin-1-yl)cyclohe xyl)-2-(dimethylamino)-4-methylpentanamide (93);
(S)-2-amino-N-(4-(4-amino-(4-phenoxyphenyl)-1H-pyrazolo[3,4-d]pyrimidin-1-yl )cyclohexyl)-4-methylpentanamide (94);
(S)-N-(4-(4-amino-(4-phenoxyphenyl)-1H-pyrazolo[3,4-d]pyrimidin-1-yl)cyclohex yl)-4-methyl-2-(methylamino)pentanamide hydrochloride (95);
(S)-N-(4-(4-amino-(4-phenoxyphenyl)-1H-pyrazolo[3,4-d]pyrimidin-1-yl)cyclohex yl)- 2-(dimethylamino)-4-methylpentanamide (96);
(R)-2-amino-N-(4-(4-amino-(4-phenoxyphenyl)-1H-pyrazolo[3,4-d]pyrimidin-1-yl )cyclohexyl)-butyramide hydrochloride (97);
(R)-N-(4-(4-amino-(4-phenoxyphenyl)-1H-pyrazolo[3,4-d]pyrimidin-1-yl)cyclohe xyl)-2-(methylamino)-butyramide hydrochloride (98);
(R)-N-(4-(4-amino-(4-phenoxyphenyl)-1H-pyrazolo[3,4-d]pyrimidin-1-yl)cyclohe xyl)-2-(dimethylamino)-butyramide (99);
(S)-2-amino-N-(4-(4-amino-(4-phenoxyphenyl)-1H-pyrazolo[3,4-d]pyrimidin-1-yl )cyclohexyl)-butyramide hydrochloride (100);
(S)-N-(4-(4-amino-(4-phenoxyphenyl)-1H-pyrazolo[3,4-d]pyrimidin-1-yl)cyclohex yl)-2-(methylamino)butyramide hydrochloride (101);
(S)-N-(4-(4-amino-(4-phenoxyphenyl)-1H-pyrazolo[3,4-d]pyrimidin-1-yl)cyclohex yl)-2-(dimethylamino)butyramide (102);
(R)-2-amino-N-(4-(4-amino-(4-phenoxyphenyl)-1H-pyrazolo[3,4-d]pyrimidin-1-yl )cyclohexyl)-pentanamide hydrochloride (103);
(R)-N-(4-(4-amino-(4-phenoxyphenyl)-1H-pyrazolo[3,4-d]pyrimidin-1-yl)cyclohe xyl)-2-(methylamino)-pentanamide hydrochloride (104);
(R)-N-(4-(4-amino-(4-phenoxyphenyl)-1H-pyrazolo[3,4-d]pyrimidin-1-yl)cyclohe xyl)-2-(dimethylamino)-pentanamide (105);
(S)-2-amino-N-(4-(4-amino-(4-phenoxyphenyl)-1H-pyrazolo[3,4-d]pyrimidin-1-yl )cyclohexyl)-pentanamide hydrochloride (106);
(S)-N-(4-(4-amino-(4-phenoxyphenyl)-1H-pyrazolo[3,4-d]pyrimidin-1-yl)cyclohex yl)-2-(methylamino)-pentanamide hydrochloride (107);
(S)-N-(4-(4-amino-(4-phenoxyphenyl)-1H-pyrazolo[3,4-d]pyrimidin-1-yl)cyclohex yl)-2-(dimethylamino)-pentanamide (108);
(R)-2-amino-N-(4-(4-amino-3-(4-phenoxyphenyl)-1H-pyrazolo[3,4-d]pyrimidin-1-yl)cis-1,4-cyclohexyl)-4-methyl-2-(methylamino)pentanamide hydrochloride (109);
(R)-2-amino-N-(4-(4-amino-3-(4-phenoxyphenyl)-1H-pyrazolo[3,4-d]pyrimidin-1-yl)cis-1,4-cyclohexyl)-4-methyl-2-(dimethylamino)pentanamide (110);
(S)-2-amino-N-(4-(4-amino-3-(4-phenoxyphenyl)-1H-pyrazolo[3,4-d]pyrimidin-1-yl)cis-1,4-cyclohexyl)-4-methylpentanamide hydrochloride (111);
(S)-2-amino-N-(4-(4-amino-3-(4-phenoxyphenyl)-1H-pyrazolo[3,4-d]pyrimidin-1-yl)cis-1,4-cyclohexyl)-4-methyl-2-(methylamino)pentanamide hydrochloride (112);
(S)-2-amino-N-(4-(4-amino-3-(4-phenoxyphenyl)-1H-pyrazolo[3,4-d]pyrimidin-1-yl)cis-1,4-cyclohexyl)-4-methyl-2-(dimethylamino)pentanamide (113);
(S)-2-amino-N-(4-(4-amino-3-(4-phenoxyphenyl)-1H-pyrazolo[3,4-d]pyrimidin-1-yl)trans-1,4-cyclohexyl)-4-methylpentanamide hydrochloride (114);
(S)-2-amino-N-(4-(4-amino-3-(4-phenoxyphenyl)-1H-pyrazolo[3,4-d]pyrimidin-1-yl)trans-1,4-cyclohexyl)-4-methyl-2-(methylamino)pentanamide hydrochloride (115);
(S)-2-amino-N-(4-(4-amino-3-(4-phenoxyphenyl)-1H-pyrazolo[3,4-d]pyrimidin-1-yl)trans-1,4-cyclohexyl)-4-methyl-2-(dimethylamino)pentanamide (116);
(R)-2-amino-N-(4-(4-amino-3-(4-phenoxyphenyl)-1H-pyrazolo[3,4-d]pyrimidin-1-yl)trans-1,4-cyclohexyl)-4-methyl-2-(methylamino)pentanamide hydrochloride (117);
(R)-2-amino-N-(4-(4-amino-3-(4-phenoxyphenyl)-1H-pyrazolo[3,4-d]pyrimidin-1-yl)trans-1,4-cyclohexyl)-4-methyl-2-(dimethylamino)pentanamide (118);
(R)-2-amino-N-(4-(4-amino-3-(4-phenoxyphenyl)-1H-pyrazolo[3,4-d]pyrimidin-1-yl)cis-1,4-cyclohexyl)-2-(5-(tert-butyl)isoxazol-3-yl)acetamide hydrochloride (119);
(R)-2-amino-N-(4-(4-amino-3-(4-phenoxyphenyl)-1H-pyrazolo[3,4-d]pyrimidin-1-yl)cis-1,4-cyclohexyl)-2-(5-(tert-butyl)isoxazol-3-yl)-2-(methylamino)acetamide hydrochloride (120);
(R)-2-amino-N-(4-(4-amino-3-(4-phenoxyphenyl)-1H-pyrazolo[3,4-d]pyrimidin-1-yl)cis-1,4-cyclohexyl)-2-(5-(tert-butyl)isoxazol-3-yl)-2-(dimethylamino)acetamide (121);
N-(4-(4-amino-3-(4-phenoxyphenyl)-1H-pyrazolo[3,4-d]pyrimidin-1-yl)cis-1,4-cy clohexyl)-2-(5-(tert-butyl)isoxazol-3-yl)acetamide (122);
N-(4-(4-amino-3-(4-phenoxyphenyl)-1H-pyrazolo[3,4-d]pyrimidin-1-yl)trans-1,4-c yclohexyl)-2-(5-(tert-butyl)isoxazol-3-yl)acetamide (123);
(S)-2-amino-N-(4-(4-amino-3-(4-phenoxyphenyl)-1H-pyrazolo[3,4-d]pyrimidin-1-yl)trans-1,4-cyclohexyl)-2-(5-(tert-butyl)isoxazol-3-yl)acetamide hydrochloride (124);
(S)-2-amino-N-(4-(4-amino-3-(4-phenoxyphenyl)-1H-pyrazolo[3,4-d]pyrimidin-1-yl)trans-1,4-cyclohexyl)-2-(5-(tert-butyl)isoxazol-3-yl)-2-(methylamino)acetamide hydrochloride (125); and
(S)-2-amino-N-(4-(4-amino-3-(4-phenoxyphenyl)-1H-pyrazolo[3,4-d]pyrimidin-1-yl)trans-1,4-cyclohexyl)-2-(5-(tert-butyl)isoxazol-3-yl)-2-(dimethylamino)acetamide (126).

9. Use of the compound or a pharmaceutically acceptable salt, solvate, isomer, ester, acid, metabolite, or prodrug thereof of any one of claims 1 to 8, wherein the hypersensitivity reactions or autoimmune inflammatory-related diseases are RIPK2 related diseases.

10. Use of the compound or a pharmaceutically acceptable salt, solvate, isomer, ester, acid, metabolite, or prodrug thereof of claim 9, wherein the hypersensitivity reactions or autoimmune inflammatory-related diseases are selected from the group consisting of: allergic rhinitis, asthma, atopic dermatitis, rheumatoid arthritis, multiple sclerosis, systemic lupus erythematosus, lupus nephritis, psoriasis, immune thrombocytopenic purpura, inflammatory bowel disease, colitis, chronic obstructive pulmonary disease, Sjögren's syndrome, pemphigus vulgaris, idiopathic plasmacytic lymphadenopathy, atherosclerosis, myocardial infarction, and thrombosis, and combinations thereof.
